# EUROPEAN PATENT SPECIFICATION

(11) **EP 3 813 777 B1**
(45) Date of publication and mention of the grant of the patent: **18.02.2026**
(21) Application number: 19732969.1
(22) Date of filing: 19.06.2019
(51) Int. Cl.: A61K 8/39, A61K 8/44, A61K 8/46, A61Q 5/02, A61K 8/60, A61Q 5/12, A61K 8/73, A61K 8/81

(54) **COMPOSITION COMPRISING AT LEAST TWO DIFFERENT ANIONIC SURFACTANTS, NON-IONIC AND AMPHOTERIC SURFACTANTS, AND CATIONIC OR AMPHOTERIC POLYMERS**
ZUSAMMENSETZUNG MIT MINDESTENS ZWEI VERSCHIEDENEN ANIONISCHEN TENSIDEN, NICHTIONISCHEN UND AMPHOTEREN TENSIDEN UND KATIONISCHEN ODER AMPHOTEREN POLYMEREN
COMPOSITION COMPRENANT AU MOINS DEUX TENSIOACTIFS ANIONIQUES DIFFÉRENTS, DES TENSIOACTIFS NON IONIQUES ET AMPHOTÈRES, ET DES POLYMÈRES CATIONIQUES OU AMPHOTÈRES

(30) Priority: 28.06.2018 FR 1855863
(43) Date of publication of application: 05.05.2021
(73) Proprietor: L'OREAL, 75008 Paris (FR)
(72) Inventor: LE CHAUX, Virginie, 93400 SAINT-OUEN (FR); MALOUG, Saber, 93400 SAINT-OUEN (FR); MATHONNEAU, Estelle, 93400 SAINT-OUEN (FR)
(74) Representative: L'Oreal
(86) International application number: PCT/EP2019/066262
(87) International publication number: WO 2020/002107

(56) References cited:
- EP-A1- 1 504 746
- WO-A1-2015/122371
- WO-A1-98/00499
- WO-A2-2007/108883
- DE-U1- 29 910 159
- FR-A1- 3 040 301
- FR-A1- 3 052 057
- STAUDIGEL J A ET AL: "Use of quaternized cassia galactomannan for hair conditioning", JOURNAL OF COSMETIC SCI, SOCIETY OF COSMETIC CHEMISTS, NEW YORK, NY, US, vol. 58, no. 6, 1 January 2007 (2007-01-01), pages 637 - 650, XP009096951, ISSN: 1525-7886
- CAROLE LEPILLEUR ET AL: "Use of statistical modeling to predict the effect of formulation composition on coacervation, silicone deposition, and conditioning sensory performance of cationic cassia polymers", JOURNAL OF COSMETIC SCIENCE, 1 March 2011 (2011-03-01), United States, pages 161 - 177, XP055085270, Retrieved from the Internet <URL:http://www.ncbi.nlm.nih.gov/pubmed/21635845> [retrieved on 20181128]

## Description

The present invention relates to a cosmetic composition, in particular a hair composition, and most particularly for cleansing and/or conditioning the hair, comprising a particular combination of anionic, nonionic and amphoteric surfactants, and also one or more cationic and/or amphoteric polymers. The invention also relates to a process for the cosmetic treatment of keratin materials using said composition, and also to the use of said composition for the cosmetic treatment of keratin materials, in particular for conferring conditioning properties on said keratin materials.

Hair can generally be damaged and embrittled by the action of external atmospheric agents such as light and bad weather, and/or by mechanical or chemical treatments, such as brushing, combing, dyeing, bleaching, permanent-waving and/or relaxing. To remedy these drawbacks, it is now common practice to have recourse to hair compositions which make it possible to condition the hair after these treatments to give it in particular sheen, softness, suppleness, lightness, a natural feel and disentangling properties. These hair compositions may be, for example, care compositions, to be applied before or after a shampooing operation, or else compositions that are both washing and conditioning compositions, such as conditioning shampoos. The latter generally have good washing power, but the cosmetic properties provided still have room for improvement; conditioning agents, most commonly silicone-based conditioning agents, are then generally included in these shampoos.

However, silicones are not always well liked by consumers, who are increasingly in search of compositions which can cleanse the hair while at the same time conditioning it, but which do not comprise silicones.

It has been proposed to replace silicones with various carbon-based conditioning agents, such as fatty alcohols or fatty esters; however, these hair compositions are not necessarily entirely satisfactory and still have room for improvement, notably with regard to the deposition of the conditioning agents, notably nonsilicone conditioning agents, on keratin fibers.

It is known from WO2015/122371, cleansing compositions comprising (A) as anionic surfactants, at least one acyl glutamic acid or its salt, at least one alkyl ether carboxylic acid or its salt, and at least one alkyl sulfosuccinic acid or its salt; (B) amphoteric surfactant, and (C) nonionic surfactant.

It is also known from EP1504746, aqueous conditioning shampoo comprising a foaming surfactant and cetyl-PEG/PPG-10/1 dimethicone.

Further, DE29910159U1 describes water-based shampoos containing C8-C22 acylaminocarboxylic acids, additional anionic surfactants of the sulfate, sulfonate, carboxylate and/or alkyl phosphate type, and glycerol mono-C10-C18 fatty acid esters.

FR3040301 describes compositions comprising anionic surfactants, at least 2% by weight of amphoteric surfactants, cationic polymers having a cationic charge density greater than or equal to 4 meq/g, and at least 3% of a mixture of specific nonionic surfactants.

There is thus a real need to develop cosmetic compositions, notably hair compositions, intended for cleansing and conditioning keratin materials, in particular the hair, which are capable of giving the hair improved cosmetic properties, notably in terms of disentangling, smooth feel and visual smoothness, suppleness and coating of the fiber, while at the same time retaining good washing power, notably good foaming power (abundant foam, rapidly generated, of good quality).

This aim is achieved by the present invention, one subject of which is a cosmetic composition, preferably a hair composition, comprising:
(i) one or more anionic surfactants chosen from polyoxyalkylenated alkyl(amido)ether carboxylic acids and salts thereof;
(ii) one or more non-sulfated anionic surfactants other than the polyoxyalkylenated alkyl(amido)ether carboxylic acid anionic surfactant(s) (i);
(iii) one or more nonionic surfactants chosen from alkyl(poly)glycosides;
(iv) one or more amphoteric surfactants;
(v) one or more polymers chosen from amphoteric polymers and cationic polymers; the composition having a weight ratio of the total content of amphoteric surfactants (iv) to the total content of non-sulfated anionic surfactants (ii) other than the polyoxyalkylenated alkyl(amido)ether carboxylic acid anionic surfactants (i) of greater than or equal to 0.5.

It has been found that the composition according to the invention has satisfactory foaming power. It allows the production of an abundant, rapidly-generated foam, which spreads easily on keratin fibers and is easy to remove on rinsing.

Certain users of shampoos, notably conditioning shampoos, may have more or less sensitized hair, i.e. hair that is generally damaged or embrittled by the action of external atmospheric agents such as light and bad weather, and/or the action of mechanical or chemical treatments such as brushing, combing, dyeing, bleaching, permanent-waving and/or relaxing. Thus, the composition according to the invention also makes it possible to improve the cosmetic properties given to the keratin fibers, notably to the hair, preferably sensitized hair. In particular, the composition according to the invention makes it possible to improve the disentangling and the suppleness of the hair, and also the smooth feel and visual smoothness of the hair, and the coating of the fibers. These properties make it possible to confer a good level of perception of care given to the hair.

The composition according to the invention also has the advantage of being stable on storage both at room temperature (20-25°C) and at 45°C, notably as regards its visual aspect and/or its viscosity.

For the purposes of the present invention, the term "stable" refers to a composition which, after two months of storage, shows no change in appearance, color, odor or viscosity.

Preferably, the composition according to the invention is noncoloring.

According to the present invention, the term "noncoloring composition" refers to a composition not containing any dye for keratin fibers, such as direct dyes or oxidation dye precursors (bases and/or couplers). If they are present, their content does not exceed 0.005% by weight, relative to the total weight of the composition. Specifically, at such a content, only the composition would be dyed, i.e. no dyeing effect would be observed on the keratin fibers.

In the following text, and unless otherwise indicated, the limits of a range of values are included in that range, notably in the expressions "between" and "ranging from ... to ...".

Moreover, the expression "at least one" used in the present description is equivalent to the expression "one or more", and may be replaced therewith.

### Anionic surfactants

As indicated above, the composition according to the invention comprises at least one anionic surfactant of polyoxyalkylenated alkyl(amido)ether carboxylic acid type (i) and at least one non-sulfated anionic surfactant (ii) other than the anionic surfactant(s) (i).

Thus, for the purposes of the invention, the cosmetic composition comprises at least two different anionic surfactants.

The term "anionic surfactant" means a surfactant including, as ionic or ionizable groups, only anionic groups.

In the present description, a species is termed "anionic" when it bears at least one permanent negative charge or when it can be ionized into a negatively charged species, under the conditions of use of the composition of the invention (for example the medium or the pH) and not comprising any cationic charge.

### Polyoxyalkylenated alkyl(amido)ether carboxylic acids (i)

The composition of the invention contains at least one anionic surfactant chosen from polyoxyalkylenated alkyl(amido)ether carboxylic acids and salts thereof, in particular those including from 2 to 50 alkylene oxide and in particular ethylene oxide groups.

The polyoxyalkylenated alkyl(amido)ether carboxylic acids that may be used are preferably chosen from those of formula (1): 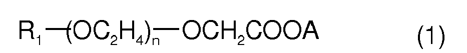 in which:
- R1 represents a linear or branched C6-C24 alkyl or alkenyl radical, a (C8-C9)alkylphenyl radical, a radical R2CONH-CH2-CH2- with R2 denoting a linear or branched C9-C21 alkyl or alkenyl radical; preferably, R1 is a C8-C20, preferably C8-C18, alkyl radical,
- n is an integer or decimal number (mean value) ranging from 2 to 24 and preferably from 2 to 10,
- A denotes H, ammonium, Na, K, Li, Mg, Ca or a monoethanolamine or triethanolamine residue.

It is also possible to use mixtures of compounds of formula (1), in particular mixtures of compounds containing different groups R1.

The polyoxyalkylenated alkyl(amido) ether carboxylic acids that are particularly preferred are those of formula (1) in which:
- R1 denotes a C₁₂-C₁₄ alkyl, cocoyl, oleyl, nonylphenyl or octylphenyl radical,
- A denotes a hydrogen or sodium atom, and
- n ranges from 2 to 20, preferably from 2 to 10.

Even more preferentially, use is made of compounds of formula (1) in which R denotes a C₁₂ alkyl radical, A denotes a hydrogen or sodium atom and n ranges from 2 to 10.

Use is preferably made of polyoxyalkylenated (C₆-C₂₄)alkyl ether carboxylic acids and salts thereof, polyoxyalkylenated (C₆-C₂₄)alkylamido ether carboxylic acids, in particular those including from 2 to 15 alkylene oxide groups, salts thereof, and mixtures thereof.

When the anionic surfactant is in salt form, said salt may be chosen from alkali metal salts, such as the sodium or potassium salt, ammonium salts, amine salts and in particular amino alcohol salts, and alkaline-earth metal salts, such as the magnesium salt.

Examples of amino alcohol salts that may be mentioned include monoethanolamine, diethanolamine and triethanolamine salts, monoisopropanolamine, diisopropanolamine or triisopropanolamine salts, 2-amino-2-methyl-1-propanol salts, 2-amino-2-methyl-1,3-propanediol salts and tris(hydroxymethyl)aminomethane salts.

Alkali metal or alkaline-earth metal salts and in particular the sodium or magnesium salts are preferably used.

Among the commercial products that may preferably be used are the products sold by the company KAO under the names:
Akypo^{®} NP 70 (R₁ = nonylphenyl, n = 7, A = H)
Akypo^{®} NP 40 (R₁ = nonylphenyl, n = 4, A = H)
Akypo^{®} OP 40 (R₁ = octylphenyl, n = 4, A = H)
Akypo^{®} OP 80 (R₁ = octylphenyl, n = 8, A = H)
Akypo^{®} OP 190 (R₁ = octylphenyl, n = 19, A = H)
Akypo^{®} RLM 38 (R₁ = (C₁₂-C₁₄)alkyl, n = 4, A = H)
Akypo^{®} RLM 38 NV (R₁ = (C₁₂-C₁₄)alkyl, n = 4, A = Na)
Akypo^{®} RLM 45 CA (R₁ = (C₁₂-C₁₄)alkyl, n = 4.5, A = H)
Akypo^{®} RLM 45 NV (R₁ = (C₁₂-C₁₄)alkyl, n = 4.5, A = Na)
Akypo^{®} RLM 100 (R₁ = (C₁₂-C₁₄)alkyl, n = 10, A = H)
Akypo^{®} RLM 100 NV (R₁ = (C₁₂-C₁₄)alkyl, n = 10, A = Na)
Akypo^{®} RLM 130 (R₁ = (C₁₂-C₁₄)alkyl, n = 13, A = H)
Akypo^{®} RLM 160 NV (R₁ = (C₁₂-C₁₄)alkyl, n = 16, A = Na),
or by the company Sandoz under the names:
   Sandopan DTC-Acid (R₁ = (C₁₃)alkyl, n = 6, A = H)
   Sandopan DTC (R₁ = (C₁₃)alkyl, n = 6, A = Na)
   Sandopan LS 24 (R₁ = (C₁₂-C₁₄)alkyl, n = 12, A = Na)
   Sandopan JA 36 (R₁ = (C₁₃)alkyl, n = 18, A = H),
   and more particularly the products sold under the following names:
      Akypo^{®} RLM 45 (INCI: Laureth-5 carboxylic acid)
      Akypo^{®} RLM 100
      Akypo^{®} RLM 38.

Polyoxyalkylenated (C₆-C₂₄)alkyl ether carboxylic acids and salts thereof are preferably used.

The composition according to the invention preferably comprises said polyoxyalkylenated alkyl(amido)ether carboxylic acid(s) and/or salts thereof in a total amount ranging from 0.1% to 30% by weight, preferably from 0.5% to 25% by weight, better still from 1% to 20% by weight and preferentially from 1.5% to 10% by weight, relative to the total weight of the composition.

### Additional non-sulfated anionic surfactants (ii)

As indicated above, the composition according to the invention comprises at least one additional anionic surfactant other than the polyoxyalkylenated alkyl(amido)ether carboxylic acids and salts thereof (i) described above. This surfactant is non-sulfated, i.e. it does not comprise any sulfate groups (OSO₃H or -OSO₃-) in its structure.

Preferably, the additional anionic surfactants (ii) used in the composition according to the invention are chosen from anionic surfactants including in their structure one or more sulfonate and/or phosphate and/or carboxylate groups, preferably sulfonate groups.

The anionic surfactant(s) (ii) may be oxyethylenated and/or oxypropylenated. The total mean number of ethylene oxide (EO) and/or propylene oxide (PO) groups may then range from 1 to 50 and notably from 1 to 10.

Among the carboxylic anionic surfactants (thus surfactants including at least one carboxylic or carboxylate function in their structure) that may be used, mention may be made of: acylglycinates, acyllactylates, acylsarcosinates, acylglutamates, alkyl-D-galactoside-uronic acids, and also the salts of these compounds; the alkyl and/or acyl groups of these compounds including from 6 to 30 carbon atoms, in particular from 12 to 28, better still from 14 to 24 or even from 16 to 22 carbon atoms; these compounds possibly being polyoxyalkylenated, notably polyoxyethylenated, and then preferably including from 1 to 50 ethylene oxide units, better still from 1 to 10 ethylene oxide units.

Mention may also be made of C6-C24 alkyl monoesters of polyglycoside-polycarboxylic acids, such as C6-C24 alkyl polyglycoside-citrates, C6-C24 alkyl polyglycoside-tartrates and C6-C24 alkyl polyglycoside-sulfosuccinates, and salts thereof.

Preferentially, the carboxylic anionic surfactants are chosen, alone or as a mixture, from:
- acylglutamates, notably of C6-C24 or even C12-C20, such as stearoylglutamates, and in particular disodium stearoylglutamate;
- acylsarcosinates, notably of C6-C24 or even C12-C20, such as palmitoylsarcosinates, and in particular sodium palmitoylsarcosinate;
- acyllactylates, notably of C12-C28 or even C14-C24, such as behenoyllactylates, and in particular sodium behenoyllactylate;
- C6-C24 and notably C12-C20 acylglycinates;
in particular in the form of alkali metal or alkaline-earth metal, ammonium or amino alcohol salts.

Among the sulfonate anionic surfactants (thus surfactants including at least one sulfonate function in their structure) that may be used, mention may be made of: alkylsulfonates, alkylamidesulfonates, alkylarylsulfonates, α-olefin sulfonates, paraffin sulfonates, alkylsulfosuccinates, alkyl ether sulfosuccinates, alkylamide sulfosuccinates, alkylsulfoacetates, N-acyltaurates, acylisethionates; alkylsulfolaurates; and also the salts of these compounds; the alkyl groups of these compounds including from 6 to 30 carbon atoms, notably from 12 to 28, better still from 14 to 24 or even from 16 to 22 carbon atoms; the aryl group preferably denoting a phenyl or benzyl group; these compounds possibly being polyoxyalkylenated, notably polyoxyethylenated, and then preferably including from 1 to 50 ethylene oxide units, better still from 2 to 10 ethylene oxide units.

Preferentially, the sulfonate anionic surfactants are chosen, alone or as a mixture, from:
- C6-C24 and notably C12-C20 olefin sulfonates;
- C6-C24 and notably C12-C20 alkylsulfosuccinates, notably laurylsulfosuccinates;
- C6-C24 and notably C12-C20 alkyl ether sulfosuccinates;
- (C6-C24)acylisethionates and preferably (C12-C18)acylisethionates;
in particular in the form of alkali metal or alkaline-earth metal, ammonium or amino alcohol salts.

When the anionic surfactant is in salt form, said salt may be chosen from alkali metal salts, such as the sodium or potassium salt, ammonium salts, amine salts and in particular amino alcohol salts, and alkaline-earth metal salts, such as the magnesium or calcium salt. Examples of amino alcohol salts that may be mentioned include monoethanolamine, diethanolamine and triethanolamine salts, monoisopropanolamine, diisopropanolamine or triisopropanolamine salts, 2-amino-2-methyl-1-propanol salts, 2-amino-2-methyl-1,3-propanediol salts and tris(hydroxymethyl)aminomethane salts. Alkali metal or alkaline-earth metal salts and in particular the sodium or magnesium salts are preferably used.

Preferentially, the additional anionic surfactants (ii) are chosen, alone or as a mixture, from:
- C6-C24 and notably C12-C20 olefin sulfonates;
- C6-C24 and notably C12-C20 alkylsulfosuccinates, notably laurylsulfosuccinates;
- C6-C24 and notably C12-C20 alkyl ether sulfosuccinates;
- (C6-C24)acylisethionates and preferably (C12-C18)acylisethionates;
- C6-C24 and notably C12-C20 acylsarcosinates; notably palmitoylsarcosinates;
- C6-C24 and notably C12-C20 acylglutamates;
- C6-C24 and notably C12-C20 acylglycinates;
in particular in the form of alkali metal or alkaline-earth metal, ammonium or amino alcohol salts.

Preferably, the anionic surfactant(s) (ii) are in the form of salts, and in particular alkaline salts, notably sodium salts, ammonium salts, amine salts, including amino alcohol salts, and/or magnesium salts. These salts preferably comprise from 2 to 5 ethylene oxide groups.

Use is more preferably made of C12-C20 olefin sulfonates, such as sodium C14-C16 olefin sulfonates.

The additional non-sulfated anionic surfactant(s) (ii) may be present in the composition according to the invention in a total content ranging from 3% to 20% by weight, preferably in a content ranging from 3.5% to 18% by weight, better still from 4% to 15% by weight and even better still from 5% to 10% by weight, relative to the total weight of the composition.

According to a particular embodiment, the total content of anionic surfactants in the composition according to the invention is between 2% and 30% by weight, notably between 3% and 25% by weight, preferably between 4% and 20% by weight, and preferentially between 5% and 15% by weight, relative to the total weight of the composition.

### Nonionic surfactants (iii)

As indicated above, the composition comprises one or more nonionic surfactants chosen from alkyl(poly)glycosides.

The composition may also comprise one or more additional (optionnal) non-ionic surfactants, other than the alkyl(poly)glycoside(s).

Preferably, the composition according to the invention comprises one or more nonionic surfactants chosen from alkyl(poly)glycosides represented by the following general formula: R1O-(R2O)t-(G)v
in which:
- R1 represents a linear or branched alkyl or alkenyl radical including 6 to 24 carbon atoms and notably 8 to 18 carbon atoms, or an alkylphenyl radical of which the linear or branched alkyl radical includes 6 to 24 carbon atoms and notably 8 to 18 carbon atoms,
- R2 represents an alkylene radical including 2 to 4 carbon atoms,
- G represents a sugar unit including 5 to 6 carbon atoms,
- t denotes a value ranging from 0 to 10 and preferably from 0 to 4,
- v denotes a value ranging from 1 to 15 and preferably from 1 to 4.

Preferably, the alkyl(poly)glycoside surfactants are compounds of the formula described above in which:
- R1 denotes a linear or branched, saturated or unsaturated alkyl radical including from 8 to 18 carbon atoms,
- R2 represents an alkylene radical including 2 to 4 carbon atoms,
- t denotes a value ranging from 0 to 3 and preferably equal to 0,
- G denotes glucose, fructose or galactose, preferably glucose,
- the degree of polymerization, i.e. the value of v, possibly ranging from 1 to 15 and preferably from 1 to 4; the mean degree of polymerization more particularly being between 1 and 2.

The glucoside bonds between the sugar units are generally of 1-6 or 1-4 type and preferably of 1-4 type. Preferably, the alkyl(poly)glycoside surfactant is an alkyl(poly)glucoside surfactant. C₈/C₁₆ alkyl(poly)glucosides 1,4, and notably decyl glucosides and caprylyl/capryl glucosides, are most particularly preferred.

Among the commercial products, mention may be made of the products sold by the company Cognis under the names Plantaren^{®} (600 CS/U, 1200 and 2000) or Plantacare^{®} (818, 1200 and 2000); the products sold by the company SEPPIC under the names Oramix CG 110 and Oramix^{®} NS 10; the products sold by the company BASF under the name Lutensol GD 70, or the products sold by the company Chem Y under the name AG10 LK.

Preferably, use is made of C₈/C₁₆-alkyl(poly)glycosides 1,4, notably as an aqueous 53% solution, such as those sold by Cognis under the reference Plantacare^{®} 818 UP.

Preferentially, the nonionic surfactants are chosen, alone or as a mixture, from (C₆-C₂₄ alkyl)(poly)glycosides, and more particularly (C₈-C₁₈ alkyl)(poly)glycosides.

The composition may also comprise additional nonionic surfactants that could be chosen from:
- alcohols, α-diols and (C1-20)alkylphenols, these compounds being polyethoxylated and/or polypropoxylated and/or polyglycerolated, the number of ethylene oxide and/or propylene oxide groups possibly ranging from 1 to 100, and the number of glycerol groups possibly ranging from 2 to 30; or else these compounds comprising at least one fatty chain including from 8 to 40 carbon atoms and in particular from 16 to 30 carbon atoms; in particular, oxyethylenated alcohols including at least one saturated or unsaturated, linear or branched C8 to C40 alkyl chain, including from 1 to 100 mol of ethylene oxide, preferably from 2 to 50 and more particularly from 2 to 40 mol of ethylene oxide and including one or two fatty chains;
- condensates of ethylene oxide and propylene oxide with fatty alcohols;
- polyethoxylated fatty amides preferably containing from 2 to 30 ethylene oxide units, polyglycerolated fatty amides including on average from 1 to 5 and in particular from 1.5 to 4 glycerol groups;

- ethoxylated fatty acid esters of sorbitan, preferably containing from 2 to 40 mol of ethylene oxide;
- fatty acid esters of sucrose;
- polyoxyalkylenated, preferably polyoxyethylenated, fatty acid esters containing from 2 to 150 mol of ethylene oxide, including oxyethylenated plant oils;
- N-(C6-24 alkyl)glucamine derivatives;
- amine oxides such as (C10-14 alkyl)amine oxides or N-(C10-14 acyl)aminopropylmorpholine oxides;

Preferably, the nonionic surfactant(s) are present in the composition according to the invention in a total content ranging from 0.1% to 15% by weight, preferentially ranging from 0.2% to 10% by weight, in particular ranging from 0.5% to 8% by weight, relative to the total weight of the composition.

Preferably, the nonionic surfactant(s) of alkyl(poly)glycoside type are present in the composition according to the invention in a total content ranging from 0.1% to 15% by weight, preferentially ranging from 0.2% to 10% by weight, in particular ranging from 0.5% to 8% by weight, relative to the total weight of the composition.

### Amphoteric surfactants (iv)

The composition according to the invention also comprises one or more amphoteric surfactants.

In particular, the amphoteric or zwitterionic surfactant(s) are nonsilicone surfactants. They may notably be optionally quaternized secondary or tertiary aliphatic amine derivatives, in which the aliphatic group is a linear or branched chain including from 8 to 22 carbon atoms, said amine derivatives containing at least one anionic group, for instance a carboxylate, sulfonate, sulfate, phosphate or phosphonate group.

Mention may in particular be made of (C₈-C₂₀)alkylbetaines, (C₈-C₂₀)alkylsulfobeta-ines, (C₈-C₂₀)alkylamido(C₃-C₈)alkylbetaines and (C₈-C₂₀)alkylamido(C₆-C₈)alkyl-sulfobetaines.

Among the optionally quaternized derivatives of secondary or tertiary aliphatic amines that may be used, as defined above, mention may also be made of the compounds having the respective structures (II) and (III) below:

Rₐ-CONHCH₂CH₂-N⁺(R_{b})(R_{c})-CH₂COO⁻, M⁺, X⁻ (II)

in which:
- Rₐ represents a C₁₀ to C₃₀ alkyl or alkenyl group derived from an acid RₐCOOH preferably present in hydrolyzed coconut kernel oil, or a heptyl, nonyl or undecyl group;
- R_{b} represents a β-hydroxyethyl group; and
- R_{c} represents a carboxymethyl group;
- M⁺ represents a cationic counterion derived from an alkali metal or alkaline-earth metal, such as sodium, an ammonium ion or an ion derived from an organic amine; and
- X⁻ represents an organic or mineral anionic counterion, such as that chosen from halides, acetates, phosphates, nitrates, (C₁-C₄)alkyl sulfates, (C₁-C₄)alkyl- or (C₁-C₄)alkylarylsulfonates, in particular methyl sulfate and ethyl sulfate; or alternatively M⁺ and X⁻ are absent;

   R_{a'}-CONHCH₂CH₂-N(B)(B') (III)

   in which:
   - B represents the group -CH₂CH₂OX';
   - B' represents the group -(CH₂)_{z}Y', with z = 1 or 2;
   - X' represents the group -CH₂COOH, -CH₂-COOZ', -CH₂CH₂COOH or CH₂CH₂-COOZ', or a hydrogen atom;
   - Y' represents the group -COOH, -COOZ' or -CH₂CH(OH)SO₃H or the group CH₂CH(OH)SO₃-Z';
   - Z' represents a cationic counterion derived from an alkali metal or alkaline-earth metal, such as sodium, an ammonium ion or an ion derived from an organic amine;
   - R_{a'} represents a C₁₀ to C₃₀ alkyl or alkenyl group of an acid R_{a'}-COOH which is preferably present in coconut kernel oil or in hydrolyzed linseed oil, or an alkyl group, notably a C₁₇ group, and its iso form, or an unsaturated C₁₇ group.

These compounds are classified in the CTFA dictionary, 5th edition, 1993, under the names disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium capryloamphodipropionate, lauroamphodipropionic acid and cocoamphodipropionic acid.

By way of example, mention may be made of the cocoamphodiacetate sold by the company Rhodia under the trade name Miranol^{®} C2M Concentrate.

Use may also be made of compounds of formula (IV):

R_{a"}-NHCH(Y")-(CH₂)ₙCONH(CH₂)ₙ'-N(R_{d})(Rₑ) (IV)

in which:
- Y" represents the group -COOH, -COOZ" or -CH₂-CH(OH)SO₃H or the group CH₂CH(OH)SO₃-Z";
- R_{d} and Rₑ, independently of each other, represent a C₁ to C₄ alkyl or hydroxyalkyl radical;
- Z" represents a cationic counterion derived from an alkali metal or alkaline-earth metal, such as sodium, an ammonium ion or an ion derived from an organic amine;
- R_{a"} represents a C₁₀ to C₃₀ alkyl or alkenyl group of an acid R_{a"}-COOH which is preferably present in coconut kernel oil or in hydrolyzed linseed oil;
- n and n', independently of each other, denote an integer ranging from 1 to 3.

Among the compounds of formula (II), mention may be made of the compound classified in the CTFA dictionary under the name sodium diethylaminopropyl cocoaspartamide and sold by the company Chimex under the name Chimexane HB. These compounds may be used alone or as mixtures.

Among the amphoteric or zwitterionic surfactants, use is preferably made of (C₈-C₂₀)alkylbetaines such as cocoylbetaine, (C₈-C₂₀)alkylamido(C₃-C₈)alkylbetaines such as cocamidopropylbetaine, and mixtures thereof, and the compounds of formula (IV) such as the sodium salt of diethylaminopropyl laurylaminosuccinamate (INCI name: sodium diethylaminopropyl cocoaspartamide).

Preferentially, the amphoteric or zwitterionic surfactants are chosen from (C₈-C₂₀)alkylamido(C₃-C₈)alkylbetaines such as cocamidopropyl betaine.

Preferably, the amphoteric surfactant(s) are present in the composition according to the invention in a total content ranging from 3% to 20% by weight, preferentially in a content ranging from 3.5% to 15% by weight and better still from 4% to 10% by weight, relative to the total weight of the composition.

The compositions according to the invention have a weight ratio of the total content of amphoteric surfactants (iv) to the total content of non-sulfated anionic surfactants (ii) other than the polyoxyalkylenated alkyl(amido)ether carboxylic acid anionic surfactants (i) of greater than or equal to 0.5, preferably greater than or equal to 0.6, in particular greater than or equal to 0.8. It may thus be between 0.5 and 3.0, notably between 0.6 and 2.0, and better still between 0.7 and 1.5.

### Cationic or amphoteric polymers

The composition according to the invention also comprises one or more polymers chosen from amphoteric polymers and cationic polymers. It is, needless to say, possible to use a mixture of polymers, in particular two different polymers, both chosen from amphoteric polymers and cationic polymers.

For the purposes of the present invention, the term "cationic polymer" means any nonsilicone (not comprising any silicon atoms) polymer containing cationic groups and/or groups that can be ionized into cationic groups and not containing any anionic groups and/or groups that can be ionized into anionic groups.

For the purposes of the present invention, the term "amphoteric polymer" means any nonsilicone (not comprising any silicon atoms) polymer containing both cationic groups and/or groups that can be ionized into cationic groups and anionic groups and/or groups that can be ionized into anionic groups.

Preferably, the cationic charge density of said polymer(s) is less than or equal to 4 meq/g.

The cationic charge density of a polymer corresponds to the number of moles of cationic charges per unit mass of polymer under conditions in which it is totally ionized. It may be determined by calculation if the structure of the polymer is known, i.e. the structure of the monomers constituting the polymer and their molar proportion or weight proportion. It may also be determined experimentally by the Kjeldahl method.

The polymers may be associative or non-associative.

The term "associative polymer" refers to an amphiphilic polymer that is capable, in an aqueous medium, of reversibly combining with itself or with other molecules. It generally includes, in its chemical structure, at least one hydrophilic region or group and at least one hydrophobic region or group. In particular, the hydrophobic group may be a fatty hydrocarbon-based chain such as a linear or branched alkyl, linear or branched arylalkyl or linear or branched alkylaryl group including at least 8 carbon atoms, preferably 8 to 30 carbon atoms, better still from 12 to 24 carbon atoms.

The cationic polymers that may be used preferably have a weight-average molar mass (Mw) of between 500 and 5×10⁶ approximately and preferably between 10³ and 3×10⁶ approximately.

Among the cationic polymers, mention may be made more particularly of:
(1) homopolymers or copolymers derived from acrylic or methacrylic esters or amides and including at least one of the units having the following formula: 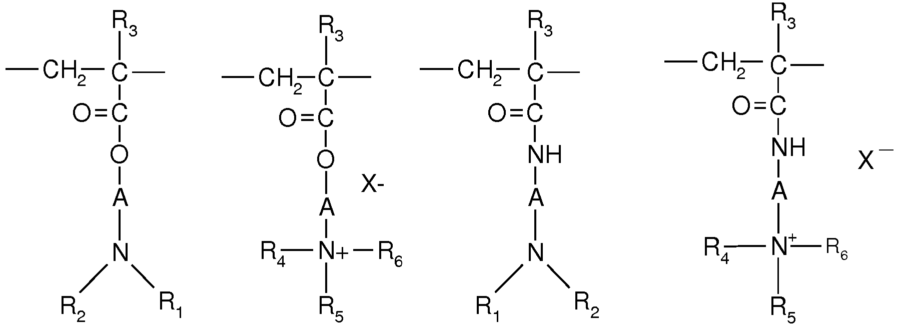 in which:
   - R3, which may be identical or different, denote a hydrogen atom or a CH3 radical;
   - A, which may be identical or different, represent a linear or branched divalent alkyl group of 1 to 6 carbon atoms, preferably 2 or 3 carbon atoms, or a hydroxyalkyl group of 1 to 4 carbon atoms;
   - R4, R5 and R6, which may be identical or different, represent an alkyl group containing from 1 to 18 carbon atoms or a benzyl radical, preferably an alkyl group containing from 1 to 6 carbon atoms;
   - R1 and R2, which may be identical or different, represent a hydrogen atom or an alkyl group containing from 1 to 6 carbon atoms, preferably methyl or ethyl;
   - X denotes an anion derived from a mineral or organic acid, such as a methosulfate anion or a halide such as chloride or bromide.

The copolymers of family (1) may also contain one or more units derived from comonomers that may be chosen from the family of acrylamides, methacrylamides, diacetone acrylamides, acrylamides and methacrylamides substituted on the nitrogen with lower (C1-C4) alkyls, acrylic or methacrylic acid esters, vinyllactams such as vinylpyrrolidone or vinylcaprolactam, and vinyl esters.

Among these copolymers of family (1), mention may be made of:
- copolymers of acrylamide and of dimethylaminoethyl methacrylate quaternized with dimethyl sulfate or with a dimethyl halide, such as that sold under the name Hercofloc by the company Hercules,
- copolymers of acrylamide and of methacryloyloxyethyltrimethylammonium chloride, such as the products sold under the name Bina Quat P 100 by the company Ciba Geigy,
- the copolymer of acrylamide and of methacryloyloxyethyltrimethylammonium methosulfate, such as that sold under the name Reten by the company Hercules,
- quaternized or non-quaternized vinylpyrrolidone/dialkylaminoalkyl acrylate or methacrylate copolymers, such as the products sold under the name Gafquat by the company ISP, for instance Gafquat 734 or Gafquat 755, or alternatively the products known as Copolymer 845, 958 and 937. These polymers are described in detail in French patents 2 077 143 and 2 393 573;
- dimethylaminoethyl methacrylate/vinylcaprolactam/vinylpyrrolidone terpolymers, such as the product sold under the name Gaffix VC 713 by the company ISP,
- vinylpyrrolidone/methacrylamidopropyldimethylamine copolymers, such as the copolymers sold under the name Styleze CC 10 by ISP;
- quaternized vinylpyrrolidone/dimethylaminopropylmethacrylamide copolymers such as the product sold under the name Gafquat HS 100 by the company ISP,
- preferably crosslinked polymers of methacryloyloxy(C1-C4)alkyltri(C1-C4)alkylammonium salts, such as the polymers obtained by homopolymerization of dimethylaminoethyl methacrylate quaternized with methyl chloride, or by copolymerization of acrylamide with dimethylaminoethyl methacrylate quaternized with methyl chloride, the homopolymerization or copolymerization being followed by crosslinking with an olefinically unsaturated compound, in particular methylenebisacrylamide. Use may be made more particularly of a crosslinked acrylamide/methacryloyloxyethyltrimethylammonium chloride copolymer (20/80 by weight) in the form of a dispersion comprising 50% by weight of said copolymer in mineral oil. This dispersion is sold under the name Salcare^{®} SC 92 by the company Ciba. Use may also be made of a crosslinked methacryloyloxyethyltrimethylammonium chloride homopolymer comprising approximately 50% by weight of the homopolymer in mineral oil or in a liquid ester. These dispersions are sold under the names Salcare^{®} SC 95 and Salcare^{®} SC 96 by the company Ciba.
(2) cationic polysaccharides, notably associative or nonassociative cationic celluloses and galactomannan gums.

Among the cationic polysaccharides, mention may be made more particularly of cellulose ether derivatives including quaternary ammonium groups, cationic cellulose copolymers or cellulose derivatives grafted with a water-soluble quaternary ammonium monomer and cationic galactomannan gums.

The cellulose ether derivatives including quaternary ammonium groups are notably described in FR 1 492 597, and mention may be made of the polymers sold under the name Ucare Polymer JR (JR 400 LT, JR 125 and JR 30M) or LR (LR 400 and LR 30M) by the company Amerchol. These polymers are also defined in the CTFA dictionary as quaternary ammoniums of hydroxyethylcellulose that have reacted with an epoxide substituted with a trimethylammonium group.

Cationic cellulose copolymers or cellulose derivatives grafted with a water-soluble quaternary ammonium monomer are described in particular in patent US 4 131 576, and mention may be made of hydroxyalkyl celluloses, for instance hydroxymethyl, hydroxyethyl or hydroxypropyl celluloses grafted, in particular, with a methacryloylethyltrimethylammonium, methacrylamidopropyltrimethylammonium or dimethyldiallylammonium salt. In particular, mention may be made of Polyquaternium 10. Among the commercial products corresponding to this definition, mention may be made of the products sold under the names Celquat L 200 and Celquat H 100 by the company National Starch.

Among the cationic polysaccharides, mention may also be made of cationic associative celluloses, or quaternized celluloses derivatives, and in particular quaternized celluloses modified with groups including at least one fatty chain, such as linear or branched alkyl groups, linear or branched arylalkyl groups, linear or branched alkylaryl groups, preferably linear or branched alkyl groups, these groups including at least 8 carbon atoms, notably from 8 to 30 carbon atoms, better still from 10 to 24 or even from 10 to 14 carbon atoms; or mixtures thereof.

Preferably, mention may be made of quaternized hydroxyethylcelluloses modified with groups including at least one fatty chain, such as linear or branched alkyl groups, linear or branched arylalkyl groups, or linear or branched alkylaryl groups, preferably linear or branched alkyl groups, these groups including at least 8 carbon atoms, notably from 8 to 30 carbon atoms, better still from 10 to 24, or even from 10 to 14, carbon atoms; or mixtures thereof.

Preferentially, mention may be made of the hydroxyethylcelluloses of formula (Ib): 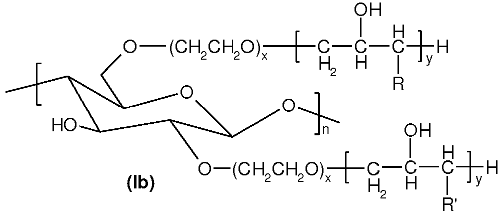 in which:
- R represents an ammonium group RaRbRcN+-, Q- in which Ra, Rb and Rc, which may be identical or different, represent a hydrogen atom or a linear or branched C1-C30 alkyl, and Q- represents an anionic counterion such as a halide, for instance a chloride or bromide; preferably an alkyl;
- R' represents an ammonium group R'aR'bR'cN+-, Q'- in which R'a, R'b and R'c, which may be identical or different, represent a hydrogen atom or a linear or branched C1-C30 alkyl, and Q'- represents an anionic counterion such as a halide, for instance a chloride or bromide; preferably an alkyl;
   it being understood that at least one of the radicals Ra, Rb, Rc, R'a, R'b and R'c represents a linear or branched C8-C30 alkyl;
- n, x and y, which may be identical or different, represent an integer between 1 and 10000.

Preferably, in formula (Ib), at least one of the radicals Ra, Rb, Rc, R'a, R'b and R'c represents a linear or branched C8-C30, better still C10-C24, or even C10-C14, alkyl; mention may be made in particular of the dodecyl radical (C12). Preferably, the other radical(s) represent a linear or branched C1-C4 alkyl, notably methyl.

Preferably, in formula (Ib), only one of the radicals Ra, Rb, Rc, R'a, R'b and R'c represents a linear or branched C8-C30, better still C10-C24, or even C10-C14, alkyl; mention may be made in particular of the dodecyl radical (C12). Preferably, the other radicals represent a linear or branched C1-C4 alkyl, notably methyl.

Even better still, R may be a group chosen from -N⁺(CH₃)₃, Q'⁻ and - N⁺(C₁₂H₂₅)(CH₃)₂, Q'⁻, preferably an -N⁺(CH₃)₃, Q'⁻ group.

Even better still, R' may be a group -N⁺(C₁₂H₂₅)(CH₃)₂, Q'⁻.

The aryl radicals preferably denote phenyl, benzyl, naphthyl or anthryl groups.

Mention may notably be made of the following polymers having the INCI names:
- Polyquaternium-24, such as the product Quatrisoft LM 200^{®}, sold by the company Amerchol/Dow Chemical;
- PG-Hydroxyethylcellulose Cocodimonium Chloride, such as the product Crodacel QM^{®};
- PG-Hydroxyethylcellulose Lauryldimonium Chloride (C12 alkyl), such as the product Crodacel QL^{®}; and
- PG-Hydroxyethylcellulose Stearyldimonium Chloride (C18 alkyl), such as the product Crodacel QS^{®}, sold by the company Croda.

Mention may also be made of the hydroxyethylcelluloses of formula (Ib) in which R represents trimethylammonium halide and R' represents dimethyldodecylammonium halide, preferentially R represents trimethylammonium chloride (CH3)3N+-, Cland **R'** represents dimethyldodecylammonium chloride (CH3)2(C12H25)N+-, CI-. This type of polymer is known under the INCI name Polyquaternium-67; as commercial products, mention may be made of the Softcat Polymer SL^{®} polymers, such as SL-100, SL-60, SL-30 and SL-5, from the company Amerchol/Dow Chemical.

More particularly, the polymers of formula (Ib) are those whose viscosity is between 2000 and 3000 cPs inclusive, preferentially between 2700 and 2800 cPs. Typically, Softcat Polymer SL-5 has a viscosity of 2500 cPs, Softcat Polymer SL-30 has a viscosity of 2700 cPs, Softcat Polymer SL-60 has a viscosity of 2700 cPs and Softcat Polymer SL-100 has a viscosity of 2800 cPs.

Among the cationic galactomannan gums, described more particularly in patents US 3 589 578 and US 4 031 307, mention may be made of guar gums comprising cationic trialkylammonium groups. Use is made, for example, of guar gums modified with a 2,3-epoxypropyltrimethylammonium salt (for example, a chloride). Such products are notably sold under the names Jaguar C13 S, Jaguar C 15, Jaguar C 17 and Jaguar C162 by the company Rhodia.

Among the cationic polysaccharides that may be used, mention may also be made of cationic derivatives of cassia gum, notably those including quaternary ammonium groups; in particular, mention may be made of the product having the INCI name Cassia hydroxypropyltrimonium chloride.
(3) polymers constituted of piperazinyl units and of divalent alkylene or hydroxyalkylene radicals containing linear or branched chains, optionally interrupted with oxygen, sulfur or nitrogen atoms or with aromatic or heterocyclic rings, and also the oxidation and/or quaternization products of these polymers.
(4) water-soluble polyaminoamides prepared in particular by polycondensation of an acidic compound with a polyamine; these polyaminoamides can be crosslinked with an epihalohydrin, a diepoxide, a dianhydride, an unsaturated dianhydride, a bis-unsaturated derivative, a bis-halohydrin, a bis-azetidinium, a bis-haloacyldiamine, a bis-alkyl halide or alternatively with an oligomer resulting from the reaction of a difunctional compound which is reactive with a bis-halohydrin, a bis-azetidinium, a bis-haloacyldiamine, a bis-alkyl halide, an epihalohydrin, a diepoxide or a bis-unsaturated derivative; the crosslinking agent being used in proportions ranging from 0.025 to 0.35 mol per amine group of the polyaminoamide; these polyaminoamides can be alkylated or, if they include one or more tertiary amine functions, they can be quaternized.
(5) polyaminoamide derivatives resulting from the condensation of polyalkylene polyamines with polycarboxylic acids followed by alkylation with difunctional agents. Mention may be made, for example, of adipic acid/dialkylaminohydroxyalkyldialkylenetriamine polymers in which the alkyl radical includes from 1 to 4 carbon atoms and preferably denotes methyl, ethyl or propyl. Among these derivatives, mention may be made more particularly of the adipic acid/dimethylaminohydroxypropyl/diethylenetriamine polymers sold under the name Cartaretine F, F4 or F8 by the company Sandoz.
(6) polymers obtained by reacting a polyalkylene polyamine including two primary amine groups and at least one secondary amine group with a dicarboxylic acid chosen from diglycolic acid and saturated aliphatic dicarboxylic acids containing from 3 to 8 carbon atoms; the mole ratio between the polyalkylene polyamine and the dicarboxylic acid preferably being between 0.8:1 and 1.4:1; the resulting polyaminoamide being reacted with epichlorohydrin in a mole ratio of epichlorohydrin relative to the secondary amine group of the polyaminoamide preferably of between 0.5:1 and 1.8:1. Polymers of this type are sold in particular under the name Hercosett 57 by the company Hercules Inc. or else under the name PD 170 or Delsette 101 by the company Hercules in the case of the adipic acid/epoxypropyl/diethylenetriamine copolymer.
(7) cyclopolymers of alkyldiallylamine or of dialkyldiallylammonium, such as the homopolymers or copolymers including, as main constituent of the chain, units corresponding to formula (I) or (II): 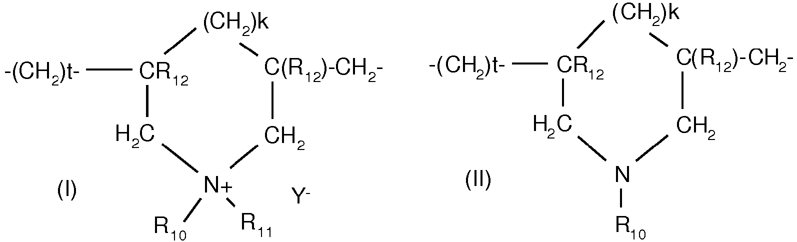 in which
   - k and t are equal to 0 or 1, the sum k + t being equal to 1;
   - R12 denotes a hydrogen atom or a methyl radical;
   - R10 and R11, independently of each other, denote a C1-C6 alkyl group, a C1-C5 hydroxyalkyl group, a C1-C4 amidoalkyl group; or alternatively R10 and R11 may denote, together with the nitrogen atom to which they are attached, a heterocyclic group such as piperidyl or morpholinyl; R10 and R11, independently of each other, preferably denote a C1-C4 alkyl group;
   - Y⁻ is an anion such as bromide, chloride, acetate, borate, citrate, tartrate, bisulfate, bisulfite, sulfate or phosphate.
   Mention may be made more particularly of the homopolymer of dimethyldiallylammonium salts (for example chloride) for example sold under the INCI name Polyquaternium-6, in particular sold under the name Merquat 100, and the copolymers of diallyldimethylammonium salts (for example chloride) and of acrylamide, for example having the INCI name Polyquaternium-7 and in particular sold under the name Merquat 550 or Merquat 7SPR.
(8) quaternary diammonium polymers comprising repeating units of formula: 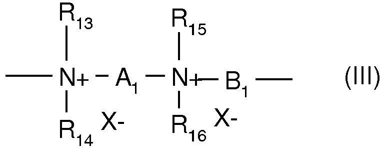 in which:
   - R13, R14, R15 and R16, which may be identical or different, represent aliphatic, alicyclic or arylaliphatic radicals comprising from 1 to 20 carbon atoms or C1-C12 hydroxyalkyl aliphatic radicals,
      or else R13, R14, R15 and R16, together or separately, form, with the nitrogen atoms to which they are attached, heterocycles optionally comprising a second nonnitrogen heteroatom;
      or else R13, R14, R15 and R16 represent a linear or branched C1-C6 alkyl radical substituted with a nitrile, ester, acyl, amide or -CO-O-R17-D or -CO-NH-R17-D group, where R17 is an alkylene and D is a quaternary ammonium group;
   - A1 and B1 represent linear or branched, saturated or unsaturated, divalent polymethylene groups comprising from 2 to 20 carbon atoms, which may contain, linked to or intercalated in the main chain, one or more aromatic rings or one or more oxygen or sulfur atoms or sulfoxide, sulfone, disulfide, amino, alkylamino, hydroxyl, quaternary ammonium, ureido, amide or ester groups, and
   - X⁻ denotes an anion derived from a mineral or organic acid;
      it being understood that A1, R13 and R15 can form, with the two nitrogen atoms to which they are attached, a piperazine ring;
      in addition, if A1 denotes a linear or branched, saturated or unsaturated alkylene or hydroxyalkylene radical, B1 may also denote a group (CH2)n-CO-D-OC-(CH2)p-with n and p, which may be identical or different, being integers ranging from 2 to 20, and D denoting:
         a) a glycol residue of formula -O-Z-O-, in which Z denotes a linear or branched hydrocarbon-based radical or a group corresponding to one of the following formulae: -(CH2CH2O)x-CH2CH2- and -[CH2CH(CH3)O]y-CH2CH(CH3)-, in which x and y denote an integer from 1 to 4, representing a defined and unique degree of polymerization or any number from 1 to 4 representing an average degree of polymerization;
         b) a bis-secondary diamine residue, such as a piperazine derivative;
         c) a bis-primary diamine residue of formula -NH-Y-NH-, in which Y denotes a linear or branched hydrocarbon-based radical, or else the divalent radical -CH2-CH2-S-S-CH2-CH2-;
         d) an ureylene group of formula -NH-CO-NH-.
   Preferably, X⁻ is an anion, such as chloride or bromide. These polymers have a number-average molar mass (Mn) generally of between 1000 and 100 000.
   Mention may be made more particularly of polymers that are constituted of repeating units corresponding to the formula: 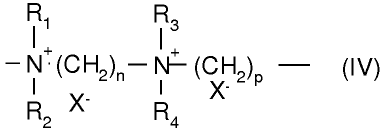 in which R1, R2, R3 and R4, which may be identical or different, denote an alkyl or hydroxyalkyl radical containing from 1 to 4 carbon atoms, n and p are integers ranging from 2 to 20, and X- is an anion derived from a mineral or organic acid.
   A particularly preferred compound of formula (IV) is the one for which R1, R2, R3 and R4 represent a methyl radical and n = 3, p = 6 and X = Cl, known as Hexadimethrine chloride according to the INCI (CTFA) nomenclature.
(9) polyquaternary ammonium polymers comprising units of formula (V): 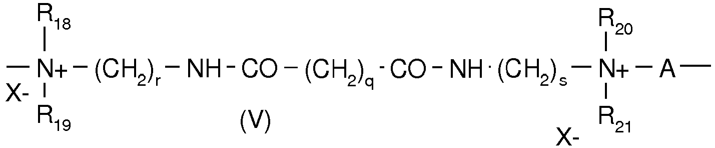 in which:
   - R18, R19, R20 and R21, which may be identical or different, represent a hydrogen atom or a methyl, ethyl, propyl, β-hydroxyethyl, β-hydroxypropyl or - CH2CH2(OCH2CH2)pOH radical, in which p is equal to 0 or to an integer between 1 and 6, with the proviso that R18, R19, R20 and R21 do not simultaneously represent a hydrogen atom,
   - r and s, which may be identical or different, are integers between 1 and 6,
   - q is equal to 0 or to an integer between 1 and 34,
   - X- denotes an anion such as a halide,
   - A denotes a divalent dihalide radical or preferably represents -CH2-CH2-O-CH2-CH2-.
   Examples that may be mentioned include the products Mirapol^{®} A 15, Mirapol^{®} AD1, Mirapol^{®} AZ1 and Mirapol^{®} 175 sold by the company Miranol.
(10) quaternary polymers of vinylpyrrolidone and of vinylimidazole, for instance the products sold under the names Luviquat^{®} FC 905, FC 550 and FC 370 by the company BASF.
(11) polyamines such as Polyquart^{®} H sold by Cognis, referred to under the name Polyethylene glycol (15) tallow polyamine in the CTFA dictionary.
(12) polymers including in their structure:
   (a) one or more units corresponding to formula (A) below: 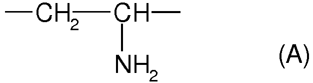
   (b) optionally one or more units corresponding to formula (B) below:

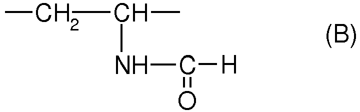

In other words, these polymers may be notably chosen from homopolymers or copolymers including one or more units derived from vinylamine and optionally one or more units derived from vinylformamide.

Preferably, these cationic polymers are chosen from polymers including, in their structure, from 5 mol% to 100 mol% of units corresponding to formula (A) and from 0 to 95 mol% of units corresponding to formula (B), preferentially from 10 mol% to 100 mol% of units corresponding to formula (A) and from 0% to 90 mol% of units corresponding to formula (B).

These polymers may be obtained, for example, by partial hydrolysis of polyvinylformamide. This hydrolysis may take place in acidic or basic medium.

The weight-average molecular weight of said polymer, measured by light scattering, may range from 1000 to 3 000 000 g/mol, preferably from 10 000 to 1 000 000 and more particularly from 100 000 to 500 000 g/mol.

The polymers including units of formula (A) and optionally units of formula (B) are notably sold under the name Lupamin by the company BASF, for instance, in a nonlimiting manner, the products provided under the names Lupamin 9095, Lupamin 5095, Lupamin 1095, Lupamin 9030 (or Luviquat 9030) and Lupamin 9010.

Other cationic polymers that may be used in the context of the invention are cationic proteins or cationic protein hydrolyzates, polyalkyleneimines, in particular polyethyleneimines, polymers comprising vinylpyridine or vinylpyridinium units, condensates of polyamines and of epichlorohydrin, quaternary polyureylenes and chitin derivatives.

Preferably, the cationic polymers are chosen from those of families (1), (2), (7) and (10) mentioned above, preferentially from those of families (2) and (7), alone or as a mixture.

The amphoteric polymers may also be chosen from amphoteric polymers comprising a repetition of:
(i) one or more units derived from a (meth)acrylamide-type monomer,
(ii) one or more units derived from a (meth)acrylamidoalkyltrialkylammonium-type monomer, and
(iii) one or more units derived from a (meth)acrylic acid-type acid monomer.

Preferably, the units derived from a (meth)acrylamide-type monomer are units of structure (la) below: 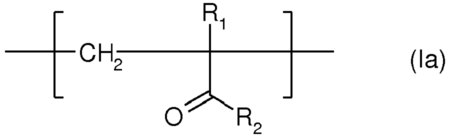 in which R₁ denotes H or CH₃ and R₂ is chosen from an amino, dimethylamino, tertbutylamino, dodecylamino and -NH-CH₂OH radical.

Preferably, said amphoteric polymer comprises a repetition of only one unit of formula (la).

The unit derived from a monomer of (meth)acrylamide type of formula (1a) in which R₁ denotes H and R₂ is an amino radical (NH2) is particularly preferred. It corresponds to the acrylamide monomer per se.

Preferably, the units derived from a monomer of (meth)acrylamidoalkyltrialkylammonium type are units of structure (IIa) below: 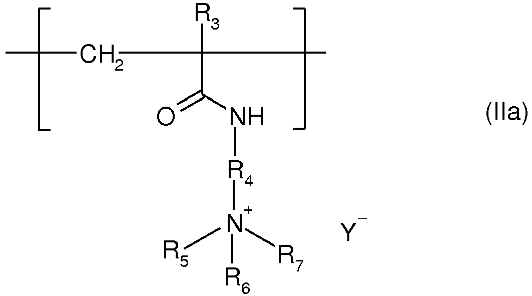 in which:
- R₃ denotes H or CH₃,
- R₄ denotes a group (CH₂)k, with k being an integer ranging from 1 to 6 and preferably from 2 to 4;
- R₅, R₆ and R₇, which may be identical or different, denote a C₁-C₄ alkyl,
- Y⁻ is an anion such as bromide, chloride, acetate, borate, citrate, tartrate, bisulfate, bisulfite, sulfate or phosphate.

Preferably, said amphoteric polymer comprises a repetition of only one unit of formula (IIa).

Among these units derived from a (meth)acrylamidoalkyltrialkylammonium-type monomer of formula (Ila), the ones that are preferred are those derived from the methacrylamidopropyltrimethylammonium chloride monomer, for which R₃ denotes a methyl radical, k is equal to 3, R₅, R₆ and R₇ denote a methyl radical, and Y⁻denotes a chloride anion.

Preferably, the units derived from a monomer of (meth)acrylic acid type are units of formula (Illa): 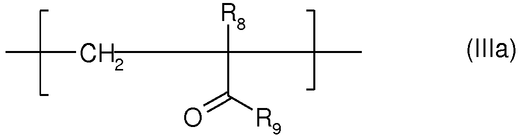 in which R₈ denotes H or CH₃ and R₉ denotes a hydroxyl radical or an -NH-C(CH₃)₂-CH₂-SO₃H radical.

The preferred units of formula (Illa) correspond to the acrylic acid, methacrylic acid and 2-acrylamido-2-methylpropanesulfonic acid monomers.

Preferably, the unit derived from a monomer of (meth)acrylic acid type of formula (Illa) is the one derived from acrylic acid, for which R₈ denotes a hydrogen atom and R₉ denotes a hydroxyl radical.

The acidic monomer(s) of (meth)acrylic acid type may be non-neutralized or partially or totally neutralized with an organic or mineral base.

Preferably, said amphoteric polymer comprises a repetition of only one unit of formula (IIIa).

According to a preferred embodiment of the invention, the amphoteric polymer(s) of this type comprise at least 30 mol% of units derived from a monomer of (meth)acrylamide type (i). Preferably, they comprise from 30 mol% to 70 mol% and more preferably from 40 mol% to 60 mol% of units derived from a (meth)acrylamidetype monomer.

The content of units derived from a monomer of (meth)acrylamidoalkyltrialkylammonium type (ii) may advantageously be from 10 mol% to 60 mol% and preferentially from 20 mol% to 55 mol%.

The content of units derived from an acidic monomer of (meth)acrylic acid type (iii) may advantageously be from 1 mol% to 20 mol% and preferentially from 5 mol% to 15 mol%.

According to a particularly preferred embodiment of the invention, the amphoteric polymer of this type comprises:
- from 30 mol% to 70 mol%, better still from 40 mol% to 60 mol%, of units derived from a monomer of (meth)acrylamide type (i),
- from 10 mol% to 60 mol%, better still from 20 mol% to 55 mol%, of units derived from a monomer of (meth)acrylamidoalkyltrialkylammonium type (ii), and
- from 1 mol% to 20 mol%, better still from 5 mol% to 15 mol%, of units derived from a monomer of (meth)acrylic acid type (iii).

Amphoteric polymers of this type may also comprise additional units, other than the units derived from a (meth)acrylamide-type monomer, a (meth)acrylamidoalkyltrialkylammonium-type monomer and a (meth)acrylic acid-type monomer such as described above.

However, according to a preferred embodiment of the invention, said amphoteric polymers are constituted solely of units derived from monomers of (meth)acrylamide type (i), of (meth)acrylamidoalkyltrialkylammonium type (ii) and of (meth)acrylic acid type (iii).

Examples of particularly preferred amphoteric polymers that may be mentioned include acrylamide/methacrylamidopropyltrimethylammonium chloride/acrylic acid terpolymers, in particular those having the INCI name: Polyquaternium-53. Corresponding products are notably sold under the names Merquat 2003 and Merquat 2003 PR by the company Nalco.

As another type of amphoteric polymer that may be used, mention may also be made of copolymers based on (meth)acrylic acid and on a dialkyldiallylammonium salt, such as copolymers of (meth)acrylic acid and of dimethyldiallylammonium chloride. An example that may be mentioned is Polyquaternium 22, notably the commercial product Merquat 280 sold by the company Nalco.

Preferably, the polymer(s) chosen from cationic polymers and/or amphoteric polymers are chosen, alone or as a mixture, from:
- cationic polysaccharides, preferentially cationic cellulose derivatives, notably cationic associative celluloses, cationic derivatives of cassia gum, notably those including quaternary ammonium groups;
- homopolymers or copolymers of dimethyldiallylammonium salts, preferentially copolymers of dimethyldiallylammonium salts, such as copolymers of diallyldimethylammonium salts (for example chloride) and of acrylamide;
- amphoteric polymers comprising:
   - from 30 mol% to 70 mol%, better still from 40 mol% to 60 mol%, of units derived from a monomer of (meth)acrylamide type (i),
   - from 10 mol% to 60 mol%, better still from 20 mol% to 55 mol%, of units derived from a monomer of (meth)acrylamidoalkyltrialkylammonium type (ii), and
   - from 1 mol% to 20 mol%, better still from 5 mol% to 15 mol%, of units derived from a monomer of (meth)acrylic acid type (iii);
notably chosen from acrylamide/methacrylamidopropyltrimethylammonium chloride/acrylic acid; in particular Polyquaternium 53.

In a particular embodiment, the composition according to the invention comprises at least:
- one cationic polysaccharide, preferably with a cationic charge density of less than or equal to 4 meq/g, notably chosen from cationic celluloses, cationic galactomannan gums and cationic cassia gums; in particular quaternary cellulose ether derivatives, cassia gum derivatives bearing quaternary ammonium groups; and
- one amphoteric polymer comprising:
   - from 30 mol% to 70 mol%, better still from 40 mol% to 60 mol%, of units derived from a monomer of (meth)acrylamide type (i),
   - from 10 mol% to 60 mol%, better still from 20 mol% to 55 mol%,of units derived from a monomer of (meth)acrylamidoalkyltrialkylammonium type (ii), and
   - from 1 mol% to 20 mol%, better still from 5 mol% to 15 mol%, of units derived from a monomer of (meth)acrylic acid type (iii);
notably chosen from acrylamide/methacrylamidopropyltrimethylammonium chloride/acrylic acid terpolymers; in particular Polyquaternium 53.

In another particular embodiment, the composition according to the invention comprises at least:
- one cationic polysaccharide, preferably with a cationic charge density of less than or equal to 4 meq/g, notably chosen from cationic celluloses, cationic galactomannan gums and cationic cassia gums; in particular quaternary cellulose ether derivatives, cassia gum derivatives bearing quaternary ammonium groups; and
- one homopolymer or copolymer of dimethyldiallylammonium salts, preferentially a copolymer of dimethyldiallylammonium salts, such as copolymers of diallyldimethylammonium salts (for example chloride) and of acrylamide.

The composition may comprise said cationic and/or amphoteric polymers in a total amount ranging from 0.01% to 5% by weight relative to the total weight of the composition, preferably from 0.05% to 4% by weight, preferentially from 0.1% to 3% by weight and better still from 0.2% to 2% by weight, relative to the total weight of the composition.

The composition according to the invention may comprise water or a mixture of water and one or more cosmetically acceptable solvents chosen from C₁-C₄ alcohols, such as ethanol, isopropanol, tert-butanol or n-butanol; polyols such as glycerol, propylene glycol and polyethylene glycols; and mixtures thereof.

Preferably, the composition according to the invention has a total water content of between 50% and 95% by weight, preferably 60% and 90% by weight, even better still between 65% and 85% by weight relative to the total weight of the composition.

The pH of the compositions according to the invention generally ranges from 3.0 to 9.0, preferably from 3.5 to 7.0, preferentially from 4.0 to 6.0 and better still from 4.5 to 5.5.

The composition according to the invention may also comprise one or more conventional additives that are well known in the art, such as natural or synthetic thickeners or viscosity regulators; C₁₂-C₃₀ fatty alcohols; ceramides; C₁₂-C₃₂ fatty esters such as isopropyl myristate, myristyl myristate, cetyl palmitate and stearyl stearate; mineral, plant or synthetic oils; vitamins or provitamins; nonionic or anionic polymers; pH stabilizers, preserving agents; dyes; fragrances; agents for preventing hair loss, antiseborrheic agents, antidandruff agents.

A person skilled in the art will take care to select the optional additives and the amount thereof such that they do not harm the properties of the compositions of the present invention.

These additives are generally present in the composition according to the invention in an amount ranging from 0 to 20% by weight relative to the total weight of the composition.

Advantageously, the composition according to the invention does not comprise any silicone (0%).

The compositions in accordance with the invention may be used as shampoos for washing and/or conditioning the hair; they are preferably applied in this case to damp hair in amounts that are effective for washing it; the lather generated by massaging or rubbing with the hands may then be removed, after an optional leave-on time, by rinsing with water, the operation possibly being repeated one or more times.

### Process and use according to the invention

Another subject of the present invention relates to a cosmetic treatment process, preferably a hair treatment process, notably for washing and/or conditioning keratin materials, in particular human keratin fibers such as the hair, comprising the application to said materials of a composition as defined above, followed by an optional leave-on time and/or rinsing and/or drying.

The composition may be applied to dry or wet hair, and preferably to wet or damp hair.

According to one embodiment, the process consists in applying to keratin fibers an effective amount of the composition according to the invention, optionally massaging the fibers, optionally leaving the composition to stand on the fibers, and rinsing. The leave-on time of the composition on the keratin fibers may be between a few seconds and 15 minutes and preferably between 30 seconds and 5 minutes. The composition is generally rinsed out with water.

An optional step of drying the keratin fibers may be performed.

The present invention also relates to the use of the composition according to the invention as described previously, for the cosmetic treatment of, notably for washing and/or conditioning, keratin materials, in particular human keratin fibers such as the hair.

The examples that follow serve to illustrate the invention without, however, being limiting in nature.

### Example 1

The cosmetic compositions (A) and (B) according to the invention and the comparative composition are prepared from the ingredients shown in the table below, the amounts of which are expressed as weight percentages of active material (AM).

| | Compositions (% AM) | | |
|---|---|---|---|
| | A | B | comparative |
| Laureth-5 carboxylic acid | 2.0 | 2.0 | 6.0 |
| Sodium C14-C16 olefin sulfonate | 7.0 | 7.0 | - |
| Cocamidopropyl betaine | 6.4 | 7.0 | 7.0 |
| Caprylyl/capryl glucoside | 1.5 | 1.5 | - |
| Cassia hydroxypropyltrimonium chloride | 0.3 | 0.3 | - |
| Polyquaternium-7 | 0.1 | - | - |
| Polyquaternium-53 | - | 0.25 | 0.25 |
| Polyquaternium-6 | - | - | 0.5 |
| Laureth-12 | - | - | 4 |
| Laureth-4 | - | - | 1 |
| Sodium laureth sulfate | - | - | 7 |
| NaCl | 1.2 | 1.3 | 1.8 |
| Preserving agents | qs | qs | qs |
| pH agent | qs pH 4.7 ± 0.3 | qs pH 4.7 ± 0.3 | qs pH 4.7 ± 0.3 |
| Water | qs 100 | qs 100 | qs 100 |

The compositions according to the invention may be used as shampoos.

It is found that compositions A and B according to the invention give the head of hair good properties in terms of disentangling, a smooth feel and visual smoothness, suppleness and coating of the fiber.

### Example 2

The cosmetic compositions C and D according to the invention are prepared from the ingredients shown in the table below, the amounts of which are expressed as weight percentages of active material (AM).

| | Compositions (% AM) | |
|---|---|---|
| | C | D |
| Laureth-5 carboxylic acid | 2.0 | 2.0 |
| Sodium C14-C16 olefin sulfonate | 7.0 | 7.0 |
| Cocamidopropyl betaine | 6.4 | 6.4 |
| Caprylyl/capryl glucoside | 1.5 | 1.5 |
| Polyquaternium-67 | 0.3 | 0.2 |
| Plant oils (coconut, soybean) | - | 0.03 |
| NaCl | 1.2 | 1.3 |
| Preserving agents | qs | qs |
| pH agent | qs pH 4.7 ± 0.3 | qs pH 5.1 ± 0.3 |
| Water | qs 100 | qs 100 |

The compositions according to the invention may be used as shampoos.

It is found that these compositions give the head of hair good properties in terms of disentangling, a smooth feel and visual smoothness, suppleness and coating of the fiber.

## Claims

1. A cosmetic composition, preferably a hair composition, comprising:
(i) one or more anionic surfactants chosen from polyoxyalkylenated alkyl(amido)ether carboxylic acids and salts thereof;
(ii) one or more non-sulfated anionic surfactants other than the polyoxyalkylenated alkyl(amido)ether carboxylic acid anionic surfactant(s) (i);
(iii) one or more nonionic surfactants chosen from alkyl(poly)glycosides;
(iv) one or more amphoteric surfactants;
(v) one or more polymers chosen from amphoteric polymers and cationic polymers; the composition having a weight ratio of the total content of amphoteric surfactants (iv) to the total content of non-sulfated anionic surfactants (ii) other than the polyoxyalkylenated alkyl(amido)ether carboxylic acid anionic surfactants (i) of greater than or equal to 0.5.

2. The composition as claimed in the preceding claim, **characterized in that** the polyoxyalkylenated alkyl(amido)ether carboxylic acids are chosen from those of formula (1): 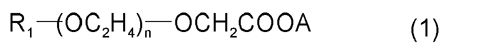 in which:
- R1 represents a linear or branched C6-C24 alkyl or alkenyl radical, a (C8-C9)alkylphenyl radical, a radical R2CONH-CH2-CH2- with R2 denoting a linear or branched C9-C21 alkyl or alkenyl radical; preferably, R1 is a C8-C20, preferably C8-C18, alkyl radical,
- n is an integer or decimal number ranging from 2 to 24 and preferably from 2 to 10,
- A denotes H, ammonium, Na, K, Li, Mg, Ca or a monoethanolamine or triethanolamine residue.

3. The composition as claimed in either of the preceding claims, comprising said polyoxyalkylenated alkyl(amido)ether carboxylic acid(s) and/or salts thereof in a total amount ranging from 0.1% to 30% by weight, preferably from 0.5% to 25% by weight, better still from 1% to 20% by weight and preferentially from 1.5% to 10% by weight, relative to the total weight of the composition.

4. The composition as claimed in one of the preceding claims, in which the non-sulfated anionic surfactants (ii) are chosen, alone or as a mixture, from:
- C6-C24 and notably C12-C20 olefin sulfonates;
- C6-C24 and notably C12-C20 alkylsulfosuccinates, notably laurylsulfosuccinates;
- C6-C24 and notably C12-C20 alkyl ether sulfosuccinates;
- (C6-C24)acylisethionates and preferably (C12-C18)acylisethionates;
- C6-C24 and notably C12-C20 acylsarcosinates; notably palmitoylsarcosinates;
- C6-C24 and notably C12-C20 acylglutamates;
- C6-C24 and notably C12-C20 acylglycinates;
in particular in the form of alkali metal or alkaline-earth metal, ammonium or amino alcohol salts.

5. The composition as claimed in one of the preceding claims, comprising the additional non-sulfated anionic surfactant(s) (ii) in a total content ranging from 3% to 20% by weight, preferably ranging from 3.5% to 18%, better still from 4% to 15% by weight and even better still from 5% to 10% by weight, relative to the total weight of the composition.

6. The composition as claimed in any one of the preceding claims, in which the alkyl(poly)glycosides are represented by the formula: R1O-(R2O)t-(G)v in which:
- R1 represents a linear or branched alkyl or alkenyl radical including 6 to 24 carbon atoms and notably 8 to 18 carbon atoms, or an alkylphenyl radical of which the linear or branched alkyl radical includes 6 to 24 carbon atoms and notably 8 to 18 carbon atoms,
- R2 represents an alkylene radical including 2 to 4 carbon atoms,
- G represents a sugar unit including 5 to 6 carbon atoms,
- t denotes a value ranging from 0 to 10 and preferably from 0 to 4,
- v denotes a value ranging from 1 to 15 and preferably 1 to 4;
in particular are (C6-C24 alkyl)(poly)glycosides, and more particularly (C8-C18 alkyl)(poly)glycosides.

7. The composition as claimed in any one of the preceding claims, comprising the nonionic surfactants in a total content ranging from 0.1% to 15% by weight, preferentially ranging from 0.2% to 10% by weight, in particular ranging from 0.5% to 8% by weight, relative to the total weight of the composition.

8. The composition as claimed in any one of the preceding claims, in which the amphoteric surfactants are chosen from (C₈-C₂₀)alkylbetaines, (C₈-C₂₀)alkylsulfobe-taines, (C₈-C₂₀)alkylamido(C₃-C₈)alkylbetaines and (C₈-C₂₀)alkylamido(C₆-C₈)alkylsulfobetaines, and also:
- the compounds having the respective structures (II) and (III) below:
Rₐ-CONHCH₂CH₂-N⁺(R_{b})(R_{c})-CH₂COO⁻, M⁺, X⁻ (II)
in which:
- Rₐ represents a C₁₀ to C₃₀ alkyl or alkenyl group derived from an acid Rₐ₋COOH preferably present in hydrolyzed coconut kernel oil, or a heptyl, nonyl or undecyl group;
- R_{b} represents a β-hydroxyethyl group; and
- R_{c} represents a carboxymethyl group;
- M⁺ represents a cationic counterion derived from an alkali metal or alkaline-earth metal, such as sodium, an ammonium ion or an ion derived from an organic amine; and
- X⁻ represents an organic or mineral anionic counterion, such as that chosen from halides, acetates, phosphates, nitrates, (C₁-C₄)alkyl sulfates, (C₁-C₄)alkyl- or (C₁-C₄)alkylarylsulfonates, in particular methyl sulfate and ethyl sulfate; or alternatively M⁺ and X⁻ are absent;
R_{a'}-CONHCH₂CH₂-N(B)(B') (III)
in which:
- B represents the group -CH₂CH₂OX';
- B' represents the group -(CH₂)_{z}Y', with z = 1 or 2;
- X' represents the group -CH₂COOH, -CH₂-COOZ', -CH₂CH₂COOH or CH₂CH₂-COOZ', or a hydrogen atom;
- Y' represents the group -COOH, -COOZ' or -CH₂CH(OH)SO₃H or the group CH₂CH(OH)SO₃-Z';
- Z' represents a cationic counterion derived from an alkali metal or alkaline-earth metal, such as sodium, an ammonium ion or an ion derived from an organic amine;
- R_{a'} represents a C₁₀ to C₃₀ alkyl or alkenyl group of an acid R_{a'}-COOH which is preferably present in coconut kernel oil or in hydrolyzed linseed oil, or an alkyl group, notably a C₁₇ group, and its iso form, or an unsaturated C₁₇ group.
- the compounds of formula (IV): R_{a"}-NHCH(Y")-(CH₂)ₙCONH(CH₂)_{n'}-N(R_{d})(Rₑ) in which:
- Y" represents the group -COOH, -COOZ" or -CH₂-CH(OH)SO₃H or the group CH₂CH(OH)SO₃-Z";
- R_{d} and Rₐ, independently of each other, represent a C₁ to C₄ alkyl or hydroxyalkyl radical;
- Z" represents a cationic counterion derived from an alkali metal or alkaline-earth metal, such as sodium, an ammonium ion or an ion derived from an organic amine;
- Rₐ represents a C₁₀ to C₃₀ alkyl or alkenyl group of an acid Rₐ-COOH which is preferably present in coconut kernel oil or in hydrolyzed linseed oil;
- n and n', independently of each other, denote an integer ranging from 1 to 3.

9. The composition as claimed in any one of the preceding claims, comprising the amphoteric surfactants in a total content ranging from 3% to 20% by weight, preferentially in a content ranging from 3.5% to 15% by weight and better still from 4% to 10% by weight relative to the total weight of the composition.

10. The composition as claimed in any one of the preceding claims, having a weight ratio of the total content of amphoteric surfactants (iv) to the total content of non-sulfated anionic surfactants (ii) other than the polyoxyalkylenated alkyl(amido)ether carboxylic acid anionic surfactants (i) of greater than or equal to 0.6, in particular greater than or equal to 0.8; preferably between 0.5 and 3.0, notably between 0.6 and 2.0, and better still between 0.7 and 1.5.

11. The composition as claimed in any one of the preceding claims, in which the cationic charge density of the polymer(s) chosen from amphoteric polymers and cationic polymers is less than or equal to 4 meq/g.

12. The composition as claimed in any one of the preceding claims, in which the polymer(s) chosen from amphoteric polymers and cationic polymers are chosen from:
(1) homopolymers or copolymers derived from acrylic or methacrylic esters or amides and including at least one of the units having the following formula: 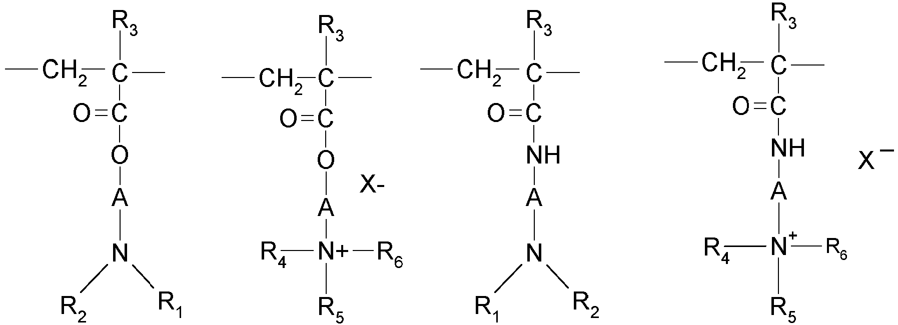 in which:
- R3, which may be identical or different, denote a hydrogen atom or a CH3 radical;
- A, which may be identical or different, represent a linear or branched divalent alkyl group of 1 to 6 carbon atoms, preferably 2 or 3 carbon atoms, or a hydroxyalkyl group of 1 to 4 carbon atoms;
- R4, R5 and R6, which may be identical or different, represent an alkyl group containing from 1 to 18 carbon atoms or a benzyl radical, preferably an alkyl group containing from 1 to 6 carbon atoms;
- R1 and R2, which may be identical or different, represent a hydrogen atom or an alkyl group containing from 1 to 6 carbon atoms, preferably methyl or ethyl;
- X denotes an anion derived from a mineral or organic acid, such as a methosulfate anion or a halide such as chloride or bromide;
(2) cationic polysaccharides, notably associative or nonassociative cationic celluloses and galactomannan gums; more particularly cellulose ether derivatives including quaternary ammonium groups, copolymers of cationic cellulose or cellulose derivatives grafted with a water-soluble quaternary ammonium monomer, cationic galactomannan gums; cationic derivatives of cassia gum, notably those including quaternary ammonium groups; and cationic associative celluloses, in particular quaternized celluloses modified with groups including at least one fatty chain, such as linear or branched alkyl groups, linear or branched arylalkyl groups, linear or branched alkylaryl groups, preferably linear or branched alkyl groups, these groups including at least 8 carbon atoms, notably from 8 to 30 carbon atoms, better still from 10 to 24 or even from 10 to 14 carbon atoms;
(3) polymers constituted of piperazinyl units and of divalent alkylene or hydroxyalkylene radicals containing linear or branched chains, optionally interrupted with oxygen, sulfur or nitrogen atoms or with aromatic or heterocyclic rings, and also the oxidation and/or quaternization products of these polymers.
(4) water-soluble polyaminoamides prepared in particular by polycondensation of an acidic compound with a polyamine; these polyaminoamides can be crosslinked with an epihalohydrin, a diepoxide, a dianhydride, an unsaturated dianhydride, a bis-unsaturated derivative, a bis-halohydrin, a bis-azetidinium, a bis-haloacyldiamine, a bis-alkyl halide or alternatively with an oligomer resulting from the reaction of a difunctional compound which is reactive with a bis-halohydrin, a bis-azetidinium, a bis-haloacyldiamine, a bis-alkyl halide, an epihalohydrin, a diepoxide or a bis-unsaturated derivative; the crosslinking agent being used in proportions ranging from 0.025 to 0.35 mol per amine group of the polyaminoamide; these polyaminoamides can be alkylated or, if they include one or more tertiary amine functions, they can be quaternized;
(5) polyaminoamide derivatives resulting from the condensation of polyalkylene polyamines with polycarboxylic acids followed by alkylation with difunctional agents;
(6) polymers obtained by reacting a polyalkylene polyamine including two primary amine groups and at least one secondary amine group with a dicarboxylic acid chosen from diglycolic acid and saturated aliphatic dicarboxylic acids containing from 3 to 8 carbon atoms;
(7) cyclopolymers of alkyldiallylamine or of dialkyldiallylammonium, such as the homopolymers or copolymers including, as main constituent of the chain, units corresponding to formula (I) or (II): 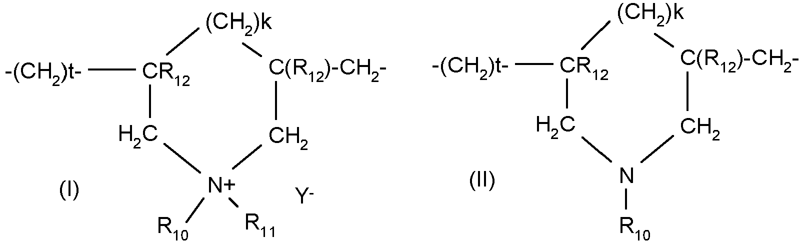 in which
- k and t are equal to 0 or 1, the sum k + t being equal to 1;
- R12 denotes a hydrogen atom or a methyl radical;
- R10 and R11, independently of each other, denote a C1-C6 alkyl group, a C1-C5 hydroxyalkyl group, a C1-C4 amidoalkyl group; or alternatively R10 and R11 may denote, together with the nitrogen atom to which they are attached, a heterocyclic group such as piperidyl or morpholinyl; R10 and R11, independently of each other, preferably denote a C1-C4 alkyl group;
- Y⁻ is an anion such as bromide, chloride, acetate, borate, citrate, tartrate, bisulfate, bisulfite, sulfate or phosphate;
(8) quaternary diammonium polymers comprising repeating units of formula: 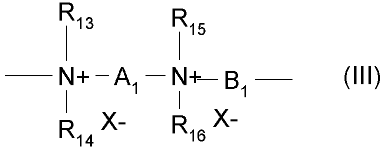 in which:
- R13, R14, R15 and R16, which may be identical or different, represent aliphatic, alicyclic or arylaliphatic radicals comprising from 1 to 20 carbon atoms or C1-C12 hydroxyalkyl aliphatic radicals,
or else R13, R14, R15 and R16, together or separately, form, with the nitrogen atoms to which they are attached, heterocycles optionally comprising a second nonnitrogen heteroatom;
or else R13, R14, R15 and R16 represent a linear or branched C1-C6 alkyl radical substituted with a nitrile, ester, acyl, amide or -CO-O-R17-D or -CO-NH-R17-D group, where R17 is an alkylene and D is a quaternary ammonium group;
- A1 and B1 represent linear or branched, saturated or unsaturated, divalent polymethylene groups comprising from 2 to 20 carbon atoms, which may contain, linked to or intercalated in the main chain, one or more aromatic rings or one or more oxygen or sulfur atoms or sulfoxide, sulfone, disulfide, amino, alkylamino, hydroxyl, quaternary ammonium, ureido, amide or ester groups, and
- X⁻ denotes an anion derived from a mineral or organic acid;
it being understood that A1, R13 and R15 can form, with the two nitrogen atoms to which they are attached, a piperazine ring;
in addition, if A1 denotes a linear or branched, saturated or unsaturated alkylene or hydroxyalkylene radical, B1 may also denote a group (CH2)n-CO-D-OC-(CH2)p-with n and p, which may be identical or different, being integers ranging from 2 to 20, and D denoting:
a) a glycol residue of formula -O-Z-O-, in which Z denotes a linear or branched hydrocarbon-based radical or a group corresponding to one of the following formulae: -(CH2CH2O)x-CH2CH2- and -[CH2CH(CH3)O]y-CH2CH(CH3)-, in which x and y denote an integer from 1 to 4, representing a defined and unique degree of polymerization or any number from 1 to 4 representing an average degree of polymerization;
b) a bis-secondary diamine residue, such as a piperazine derivative;
c) a bis-primary diamine residue of formula -NH-Y-NH-, in which Y denotes a linear or branched hydrocarbon-based radical, or else the divalent radical -CH2-CH2-S-S-CH2-CH2-;
d) an ureylene group of formula -NH-CO-NH-.
(9) polyquaternary ammonium polymers comprising units of formula (V): 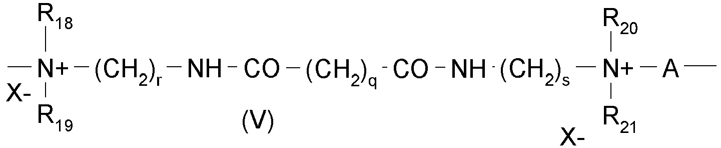 in which:
- R18, R19, R20 and R21, which may be identical or different, represent a hydrogen atom or a methyl, ethyl, propyl, β-hydroxyethyl, β-hydroxypropyl or - CH2CH2(OCH2CH2)pOH radical, in which p is equal to 0 or to an integer between 1 and 6, with the proviso that R18, R19, R20 and R21 do not simultaneously represent a hydrogen atom,
- r and s, which may be identical or different, are integers between 1 and 6,
- q is equal to 0 or to an integer between 1 and 34,
- X- denotes an anion such as a halide,
- A denotes a divalent dihalide radical or preferably represents -CH2-CH2-O-CH2-CH2-;
(10) quaternary polymers of vinylpyrrolidone and of vinylimidazole;
(11) polyamines;
(12) polymers including in their structure:
(a) one or more units corresponding to formula (A) below: 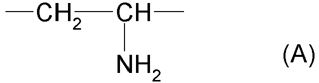
(b) optionally one or more units corresponding to formula (B) below: 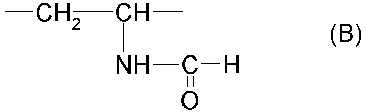
(13) amphoteric polymers comprising a repetition of:
(i) one or more units derived from a (meth)acrylamide-type monomer,
(ii) one or more units derived from a (meth)acrylamidoalkyltrialkylammonium-type monomer, and
(iii) one or more units derived from a (meth)acrylic acid-type acid monomer.

13. The composition as claimed in any one of the preceding claims, in which the polymer(s) chosen from amphoteric polymers and cationic polymers are chosen, alone or as a mixture, from:
- cationic polysaccharides, preferentially cationic cellulose derivatives, notably cationic associative celluloses, cationic derivatives of cassia gum, notably those including quaternary ammonium groups;
- homopolymers or copolymers of dimethyldiallylammonium salts, preferentially copolymers of dimethyldiallylammonium salts, such as copolymers of diallyldimethylammonium salts (for example chloride) and of acrylamide;
- amphoteric polymers comprising:
- from 30 mol% to 70 mol%, better still from 40 mol% to 60 mol%, of units derived from a monomer of (meth)acrylamide type (i),
- from 10 mol% to 60 mol%, better still from 20 mol% to 55 mol%, of units derived from a monomer of (meth)acrylamidoalkyltrialkylammonium type (ii), and
- from 1 mol% to 20 mol%, better still from 5 mol% to 15 mol%, of units derived from a monomer of (meth)acrylic acid type (iii);
notably chosen from acrylamide/methacrylamidopropyltrimethylammonium chloride/acrylic acid terpolymers; in particular Polyquaternium 53.

14. The composition as claimed in any one of the preceding claims, comprising said cationic and/or amphoteric polymers in a total amount ranging from 0.01% to 5% by weight relative to the total weight of the composition, preferably from 0.05% to 4% by weight, preferentially from 0.1% to 3% by weight and better still from 0.2% to 2% by weight, relative to the total weight of the composition.

15. The composition as claimed in any one of the preceding claims, comprising water, notably in a total content of between 50% and 95% by weight, preferably between 60% and 90% and better still between 65% and 85% by weight, relative to the total weight of the composition.

16. A cosmetic treatment process, preferably a hair treatment process, notably for washing and/or conditioning keratin materials, in particular human keratin fibers such as the hair, comprising the application to said materials of a composition as claimed in one of the preceding claims, followed by an optional leave-on time and/or rinsing and/or drying.

17. The use of the composition as claimed in one of claims 1 to 15, for the cosmetic treatment of, in particular for washing and/or conditioning, keratin materials, in particular human keratin fibers such as the hair.

## Patentansprüche

1. Kosmetische Zusammensetzung, vorzugsweise Haarzusammensetzung, umfassend:
(i) ein oder mehrere anionische Tenside, ausgewählt aus polyoxyalkylenierten Alkyl(amido)ethercarbonsäuren und Salzen davon;
(ii) ein oder mehrere nicht sulfatierte anionische Tenside, die von dem (den) anionischen polyoxyalkylenierten Alkyl(amido)ethercarbonsäuretensid(en) (i) verschieden sind;
(iii) ein oder mehrere nichtionische Tenside, ausgewählt aus Alkyl(poly)glycosiden;
(iv) ein oder mehrere amphotere Tenside;
(v) ein oder mehrere Polymere, ausgewählt aus amphoteren Polymeren und kationischen Polymeren; wobei die Zusammensetzung ein Gewichtsverhältnis des Gesamtgehalts an amphoteren Tensiden (iv) zum Gesamtgehalt an nicht sulfatierten anionischen Tensiden (ii), die von dem (den) anionischen polyoxyalkylenierten Alkyl(amido)ethercarbonsäuretensid(en) (i) verschieden sind, größer oder gleich 0,5 aufweist.

2. Zusammensetzung nach dem vorhergehenden Anspruch, **dadurch gekennzeichnet, dass** die polyoxyalkylierten Alkyl(amido)ethercarbonsäuren ausgewählt sind aus denjenigen der Formel (1):
R₁-(OC₂H₄)ₙ-OCH₂COOA (1)
wobei:
- R1 für einen linearen oder verzweigten C6-C24-Alkyloder -Alkenylrest, einen (C8-C9)-Alkylphenylrest oder einen Rest R2CONH-CH2-CH2- steht, wobei R2 für einen linearen oder verzweigten C9-C21-Alkyl- oder - Alkenylrest steht; vorzugsweise R₁ für einen C8-C20- und vorzugsweise C8-C18-Alkylrest steht,
- n eine ganze Zahl oder Dezimalzahl im Bereich von 2 bis 24 und vorzugsweise von 2 bis 10 ist,
- A H, Ammonium, Na, K, Li, Mg, Ca oder einen Monoethanolamin- oder Triethanolaminrest bedeutet.

3. Zusammensetzung nach einem der vorhergehenden Ansprüche, umfassend die polyoxyalkylenierte(n) Alkyl(amido)ethercarbonsäure(en) und/oder Salze davon in einer Gesamtmenge im Bereich von 0,1 bis 30 Gew.-%, vorzugsweise von 0,5 bis 25 Gew.-%, noch besser von 1 bis 20 Gew.-% und bevorzugt von 1,5 bis 10 Gew.-%, bezogen auf das Gesamtgewicht der Zusammensetzung.

4. Zusammensetzung nach einem der vorhergehenden Ansprüche, wobei die nicht sulfatierten anionischen Tenside (ii) allein oder als Mischung ausgewählt sind aus:
- C6-C24- und insbesondere C12-C20-Olefinsulfonaten;
- C6-C24- und insbesondere C12-C20-Alkylsulfosuccinaten, insbesondere Laurylsulfosuccinaten;
- C6-C24- und insbesondere C12-C20-Alkylethersulfosuccinaten;
- (C6-C24)-Acylisethionaten und vorzugsweise (C12-C18)-Acylisethionaten;
- C6-C24- und insbesondere C12-C20-Acylsarcosinaten; insbesondere Palmitoylsarcosinaten;
- C6-C24- und insbesondere C12-C20-Acylglutamaten;
- C6-C24- und insbesondere C12-C20-Acylglycinaten; insbesondere in Form von Alkali- oder Erdalkalimetall-, Ammonium- oder Aminoalkoholsalzen.

5. Zusammensetzung nach einem der vorhergehenden Ansprüche, umfassend das (die) zusätzliche(n) nicht sulfatierte(n) anionische(n) Tensid(e) (ii) in einem Gesamtgehalt im Bereich von 3 bis 20 Gew.-%, vorzugsweise im Bereich von 3,5 bis 18%, Gew.-%, noch besser von 4 bis 15 Gew.-% und sogar noch besser von 5 bis 10 Gew.-%, bezogen auf das Gesamtgewicht der Zusammensetzung.

6. Zusammensetzung nach einem der vorhergehenden Ansprüche, wobei die Alkyl(poly)glycoside durch die folgende Formel wiedergegeben werden: R10-(R20)t-(G)v, wobei:
- R₁ für einen linearen oder verzweigten Alkyl- oder Alkenylrest mit 6 bis 24 Kohlenstoffatomen und insbesondere 8 bis 18 Kohlenstoffatomen oder einen Alkylphenylrest, dessen linearer oder verzweigter Alkylrest 6 bis 24 Kohlenstoffatome und insbesondere 8 bis 18 Kohlenstoffatome enthält, steht;
- R2 für einen Alkylenrest mit 2 bis 4 Kohlenstoffatomen steht;
- G für eine Zuckereinheit mit 5 bis 6 Kohlenstoffatomen steht;
- t einen Wert im Bereich von 0 bis 10 und vorzugsweise von 0 bis 4 bedeutet;
- v einen Wert im Bereich von 1 bis 15 und vorzugsweise von 1 bis 4 bedeutet;
insbesondere (C6-C24-Alkyl) (poly)glycoside, und spezieller (C8-C18-Alkyl) (poly)glycoside, sind.

7. Zusammensetzung nach einem der vorhergehenden Ansprüche, umfassend die nichtionischen Tenside in einem Gesamtgehalt im Bereich von 0,1 bis 15 Gew.-%, bevorzugt im Bereich von 0,2 bis 10 Gew.-%, insbesondere im Bereich von 0,5 bis 8 Gew.-%, bezogen auf das Gesamtgewicht der Zusammensetzung.

8. Zusammensetzung nach einem der vorhergehenden Ansprüche, wobei die amphoteren Tenside ausgewählt sind aus (C₈-C₂₀) Alkylbetainen, (C₈-C₂₀) Alkylsulfobetainen, (C₈-C₂₀) Alkylamido (C₃-C₈) alkylbetainen und (C₈-C₂₀)Alkylamido(C₆-C₈)alkylsulfobetainen sowie:
- den Verbindungen mit den jeweiligen nachstehenden Strukturen (II) und (III):
Rₐ-CONHCH₂CH₂-N⁺(R_{b})(R_{c})-CH₂COO⁻, M⁺, X⁻ (II)
wobei:
- Rₐ für eine C₁₀- bis C₃₀-Alkyl- oder -Alkenylgruppe, die sich von einer Säure Rₐ-COOH ableitet, die vorzugsweise in hydrolysiertem Kokoskernöl vorliegt, oder eine Heptyl, Nonyl- oder Undecylgruppe steht;
- R_{b} für eine β-Hydroxyethylgruppe steht; und
- R_{c} für eine Carboxymethylgruppe steht;
- M⁺ für ein kationisches Gegenion, das sich von einem Alkalimetall oder Erdalkalimetall, wie Natrium, ableitet, ein Ammoniumion oder ein Ion, das sich von einem organischen Amin ableitet, steht; und
- X⁻ für ein organisches oder mineralisches anionisches Gegenion steht, wie dasjenige, das aus Halogeniden, Acetaten, Phosphaten, Nitraten, (C₁-C₄)Alkylsulfaten, (C₁-C₄)Alkyl- oder (C₁-C₄)Alkylarylsulfonaten, insbesondere Methylsulfat und Ethylsulfat, ausgewählt ist; oder alternativ dazu M⁺ und X⁻ fehlen;
R_{a'}-CONHCH₂CH₂-N(B) (B') (iii)
wobei:
- B für die Gruppe -CH₂CH₂OX' steht;
- B' für die Gruppe -(CH₂)_{z}Y' mit z = 1 oder 2 steht;
- X' für die Gruppe -CH₂COOH, -CH₂-COOZ', -CH₂CH₂COOH oder CH₂CH₂-COOZ' oder ein Wasserstoffatom steht;
- Y' für die Gruppe -COOH, -COOZ' oder -CH₂CH(OH)SO₃H oder die Gruppe CH₂CH(OH)SO₃-Z' steht;
- Z' für ein kationisches Gegenion, das sich von einem Alkalimetall oder Erdalkalimetall, wie Natrium, ableitet, ein Ammoniumion oder ein Ion, das sich von einem organischen Amin ableitet, steht;
- R_{a'} für eine C₁₀- bis C₃₀-Alkyl- oder -Alkenylgruppe einer Säure R_{a'}-COOH, die vorzugsweise in Kokoskernöl oder in hydrolysiertem Leinsamenöl vorliegt, oder eine Alkylgruppe, insbesondere eine C₁₇-Gruppe und deren Isoform oder eine ungesättigte C₁₇-Gruppe, steht;
- den Verbindungen der Formel (IV): R_{a"}-NHCH(Y")-(CH₂)ₙCONH(CH₂)ₙ-N(R_{d})(Rₑ)
wobei:
- Y" für die Gruppe -COOH, -COOZ" oder -CH₂CH(OH)SO₃H oder die Gruppe CH₂CH(OH)SO₃-Z" steht;
- R_{d} und Rₑ unabhängig voneinander für einen C₁- bis C₄-Alkyl- oder -Hydroxyalkylrest stehen;
- Z" für ein kationisches Gegenion, das sich von einem Alkalimetall oder Erdalkalimetall, wie Natrium, ableitet, ein Ammoniumion oder ein Ion, das sich von einem organischen Amin ableitet, steht;
- R_{a"} für eine C₁₀- bis C₃₀-Alkyl- oder -Alkenylgruppe einer Säure R_{a"}-COOH, die vorzugsweise in Kokoskernöl oder in hydrolysiertem Leinöl vorliegt, steht;
- n und n' unabhängig voneinander eine ganze Zahl im Bereich von 1 bis 3 bedeuten.

9. Zusammensetzung nach einem der vorhergehenden Ansprüche, umfassend die amphoteren Tenside in einem Gesamtgehalt im Bereich von 3 bis 20 Gew.-%, bevorzugt in einem Gehalt im Bereich von 3,5 bis 15 Gew.-% und noch besser von 4 bis 10 Gew.-%, bezogen auf das Gesamtgewicht der Zusammensetzung.

10. Zusammensetzung nach einem der vorhergehenden Ansprüche, mit einem Gewichtsverhältnis des Gesamtgehalts an amphoteren Tensiden (iv) zum Gesamtgehalt an nicht sulfatierten anionischen Tensiden (ii), die von dem (den) anionischen polyoxyalkylenierten Alkyl(amido)ethercarbonsäuretensid(en) (i) verschieden sind, größer oder gleich 0,6, insbesondere größer oder gleich 0,8; vorzugsweise zwischen 0,5 und 3,0, insbesondere zwischen 0,6 und 2,0 und noch besser zwischen 0,7 und 1,5.

11. Zusammensetzung nach einem der vorhergehenden Ansprüche, wobei die kationische Ladungsdichte des Polymers bzw. der Polymere, das bzw. die aus amphoteren Polymeren und kationischen Polymeren ausgewählt ist bzw. sind, kleiner oder gleich 4 meq/g ist.

12. Zusammensetzung nach einem der vorhergehenden Ansprüche, wobei das Polymer bzw. die Polymere, das bzw. die aus amphoteren Polymeren und kationischen Polymeren ausgewählt sind, ausgewählt sind aus:
(1) Homo- oder Copolymeren, die sich von Acrylsäure- oder Methacrylsäureestern oder -amiden ableiten und mindestens eine der Einheiten der folgenden Formel enthalten: 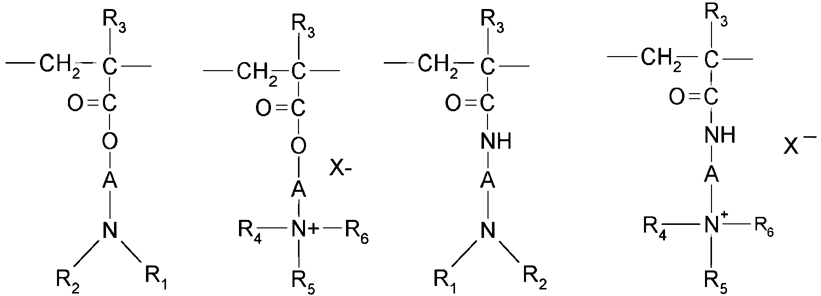 wobei:
- die Gruppen R3, die gleich oder verschieden sein können, ein Wasserstoffatom oder einen CH3-Rest bedeuten;
- die Gruppen A, die gleich oder verschieden sein können, für eine lineare oder verzweigte zweiwertige Alkylgruppe mit 1 bis 6 Kohlenstoffatomen, vorzugsweise 2 oder 3 Kohlenstoffatomen, oder eine Hydroxyalkylgruppe mit 1 bis 4 Kohlenstoffatomen stehen;
- R4, R5 und R6, die gleich oder verschieden sein können, für eine Alkylgruppe mit 1 bis 18 Kohlenstoffatomen oder einen Benzylrest, vorzugsweise eine Alkylgruppe mit 1 bis 6 Kohlenstoffatomen, stehen;
- R1 und R2, die gleich oder verschieden sein können, für ein Wasserstoffatom oder eine Alkylgruppe mit 1 bis 6 Kohlenstoffatomen, vorzugsweise Methyl oder Ethyl, stehen;
- X ein Anion, das sich von einer mineralischen oder organischen Säure ableitet, wie ein Methosulfat-Anion oder ein Halogenid, wie Chlorid oder Bromid, bedeutet;
(2) kationischen Polysacchariden, insbesondere assoziativen oder nichtassoziativen kationischen Cellulosen und Galactomannangummen; spezieller Celluloseetherderivaten mit quartären Ammoniumgruppen, Copolymeren von kationischer Cellulose oder Cellulosederivaten, die mit einem wasserlöslichen quartären Ammoniummonomer gepfropft sind, kationischen Galactomannangummen; kationischen Derivaten von Cassiagummi, insbesondere denjenigen mit quartären Ammoniumgruppen; und kationischen assoziativen Cellulosen, insbesondere quaternisierten Cellulosen, die mit Gruppen mit mindestens einer Fettkette, wie linearen oder verzweigten Alkylgruppen, linearen oder verzweigten Arylalkylgruppen, linearen oder verzweigten Alkylarylgruppen, vorzugsweise linearen oder verzweigten Alkylgruppen, modifiziert sind, wobei diese Gruppen mindestens 8 Kohlenstoffatome, insbesondere 8 bis 30 Kohlenstoffatome, noch besser 10 bis 24 oder sogar 10 bis 14 Kohlenstoffatome enthalten;
(3) Polymeren aus Piperazinyleinheiten und zweiwertigen Alkylen- oder Hydroxyalkylenresten mit linearen oder verzweigten Ketten, die gegebenenfalls durch Sauerstoff, Schwefel- oder Stickstoffatome oder durch aromatische oder heterocyclische Ringe unterbrochen sind, sowie den Oxidations- und/oder Quaternisierungsprodukten dieser Polymere.
(4) wasserlöslichen Polyaminoamiden, insbesondere hergestellt durch Polykondensation einer sauren Verbindung mit einem Polyamin; wobei diese Polyaminoamide mit einem Epihalogenhydrin, einem Diepoxid, einem Dianhydrid, einem ungesättigten Dianhydrid, einem zweifach ungesättigten Derivat, einem Bishalogenhydrin, einem Bisazetidinium, einem Bishalogenacyldiamin, einem Bisalkylhalogenid oder alternativ dazu mit einem Oligomer, das sich aus der Reaktion einer difunktionellen Verbindung, die gegenüber einem Bishalogenhydrin, einem Bisazetidinium, einem Bishalogenacyldiamin, einem Bisalkylhalogenid, einem Epihalogenhydrin, einem Diepoxid oder einem zweifach ungesättigten Derivat reaktiv ist, ergibt, vernetzt sein können; wobei das Vernetzungsmittel in Anteilen im Bereich von 0,025 bis 0,35 mol pro Amingruppe des Polyaminoamids verwendet wird; wobei diese Polyaminoamide alkyliert sein können oder, falls sie eine oder mehrere tertiäre Aminfunktionen umfassen, quaterniert sein können;
(5) Polyaminoamidderivaten, die sich aus der Kondensation von Polyalkylenpolyaminen mit Polycarbonsäuren mit anschließender Alkylierung mit difunktionellen Mitteln ergeben;
(6) Polymeren, erhalten durch Reaktion eines Polyalkylenpolyamins mit zwei primären Amingruppen und mindestens einer sekundären Amingruppe mit einer Dicarbonsäure, die aus Diglykolsäure und gesättigten aliphatischen Dicarbonsäuren mit 3 bis 8 Kohlenstoffatomen ausgewählt ist;
(7) Cyclopolymeren von Alkyldiallylamin oder Dialkyldiallylammonium, wie den Homopolymeren oder Copolymeren, die als Hauptbestandteil der Kette Einheiten der Formel (I) oder (II) entsprechende Einheiten umfassen: 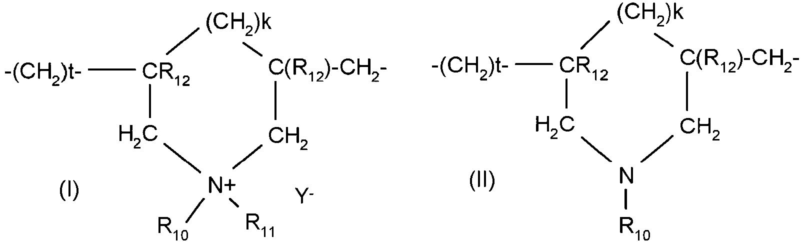 wobei
- k und t gleich 0 oder 1 sind, wobei die Summe k + t gleich 1 ist;
- R12 ein Wasserstoffatom oder einen Methylrest bedeutet;
- R10 und R11 unabhängig voneinander eine C1-C6-Alkylgruppe, eine C1-C5-Hydroxyalkylgruppe oder eine C1-C4-Amidoalkylgruppe bedeuten oder R10 und R11 alternativ dazu zusammen mit dem Stickstoffatom, an das sie gebunden sind, eine heterocyclische Gruppe wie Piperidyl oder Morpholinyl bedeuten können; wobei R10 und R11 unabhängig voneinander vorzugsweise eine C1-C4-Alkylgruppe bedeuten;
- Y⁻ ein Anion wie Bromid, Chlorid, Acetat, Borat, Citrat, Tartrat, Hydrogensulfat, Hydrogensulfit, Sulfat oder Phosphat ist;
(8) quaternären Diammoniumpolymeren, umfassend Wiederholungseinheiten der folgenden Formel: 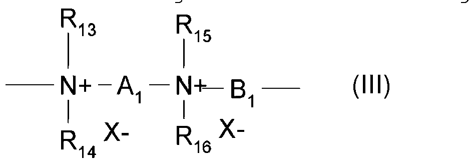 wobei:
- R13, R14, R15 und R16, die gleich oder verschieden sein können, für aliphatische, alicyclische oder arylaliphatische Reste mit 1 bis 20 Kohlenstoffatomen oder aliphatische C1-C12-Hydroxyalkylreste stehen,
oder auch R13, R14, R15 und R16 zusammen oder separat mit den Stickstoffatomen, an die sie gebunden sind, Heterocyclen bilden, die gegebenenfalls ein zweites Nicht-Stickstoff-Heteroatom umfassen;
oder auch R13, R14, R15 und R16 für einen linearen oder verzweigten C1-C6-Alkylrest stehen, der durch eine Nitril-, Ester-, Acyl-, Amid- oder -CO-0-R17-D- oder - CO-NH-R17-D-Gruppe substituiert ist, wobei R17 ein Alkylen ist und D eine quartäre Ammoniumgruppe ist;
- A1 und B1 für lineare oder verzweigte, gesättigte oder ungesättigte, zweiwertige Polymethylengruppen mit 2 bis 20 Kohlenstoffatomen stehen, die an die Hauptkette gebunden oder in die Hauptkette eingeschoben einen oder mehrere aromatische Ringe oder ein oder mehrere Sauerstoff- oder Schwefelatome oder Sulfoxid-, Sulfon-, Disulfid-, Amino-, Alkylamino-, Hydroxyl-, quartäre Ammonium-, Ureido-, Amid- oder Estergruppen enthalten können, und
- X⁻ ein Anion bedeutet, das sich von einer mineralischen oder organischen Säure ableitet;
wobei es sich versteht, dass A1, R13 und R15 mit den beiden Stickstoffatomen, an die sie gebunden sind, einen Piperazinring bilden können;
außerdem dann, wenn A1 einen linearen oder verzweigten, gesättigten oder ungesättigten Alkylen- oder Hydroxyalkylenrest bedeutet, B1 auch eine Gruppe (CH2)n-CO-D-OC-(CH2)p- bedeuten kann, wobei n und p, die gleich oder verschieden sein können, ganze Zahlen im Bereich von 2 bis 20 sind und D Folgendes bedeutet:
a) einen Glykolrest der Formel -O-Z-O-, wobei Z einen linearen oder verzweigten Rest auf Kohlenwasserstoffbasis oder eine Gruppe, die einer der folgenden Formeln entspricht, bedeutet: -(CH2CH2O)x-CH2CH2- und -[CH2CH(CH3)O]y-CH2CH(CH3)-, wobei x und y eine ganze Zahl von 1 bis 4 bedeuten und einen definierten und einzigartigen Polymerisationsgrad wiedergeben oder eine beliebige Zahl von 1 bis 4 bedeuten und einen durchschnittlichen Polymerisationsgrad wiedergeben;
b) einen bissekundären Diaminrest, wie ein Piperazinderivat;
c) einen bisprimären Diaminrest der Formel -NH-Y-NH-, wobei Y einen linearen oder verzweigten Rest auf Kohlenwasserstoffbasis oder auch den zweiwertigen Rest - CH2-CH2-S-S-CH2-CH2- bedeutet;
d) eine Ureylengruppe der Formel -NH-CO-NH-;
(9) polyquaternären Ammoniumpolymeren, umfassend Einheiten der Formel (V): 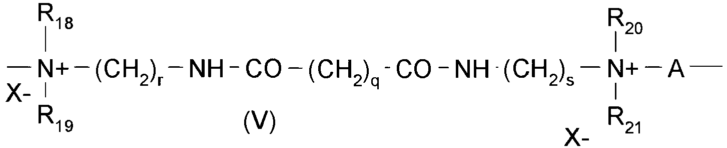 wobei:
- R18, R19, R20 und R21, die gleich oder verschieden sein können, für ein Wasserstoffatom oder einen Methyl-, Ethyl-, Propyl-, β-Hydroxyethyl-, β-Hydroxypropyl- oder -CH2CH2(OCH2CH2)pOH-Rest stehen, wobei p gleich 0 oder eine ganze Zahl zwischen 1 und 6 ist, mit der Maßgabe, dass R18, R19, R20 und R21 nicht gleichzeitig für ein Wasserstoffatom stehen,
- r und s, die gleich oder verschieden sein können, ganze Zahlen zwischen 1 und 6 sind;
- q gleich 0 oder eine ganze Zahl zwischen 1 und 34 ist,
- X- ein Anion wie ein Halogenid bedeutet,
- A einen zweiwertigen Dihalogenidrest bedeutet oder vorzugsweise für -CH2-CH2-O-CH2-CH2- steht;
(10) quaternären Polymeren von Vinylpyrrolidon und von Vinylimidazol;
(11) Polyaminen;
(12) Polymeren, die in ihrer Struktur Folgendes enthalten:
(a) eine oder mehrere Einheiten, die der nachstehenden Formel (A) entsprechen: 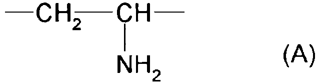
(b) gegebenenfalls eine oder mehrere Einheiten, die der nachstehenden Formel (B) entsprechen: 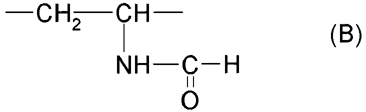
(13) amphoteren Polymeren, umfassend eine Wiederholung von:
(i) einer oder mehreren Einheiten, die sich von einem Monomer vom (Meth)acrylamid-Typ ableiten,
(ii) einer oder mehreren Einheiten, die sich von einem Monomer vom (Meth)acrylamidoalkyltrialkylammonium-Typ ableiten, und
(iii) einer oder mehreren Einheiten, die sich von einem Säuremonomer vom (Meth)acrylsäure-Typ ableiten.

13. Zusammensetzung nach einem der vorhergehenden Ansprüche, wobei das Polymer bzw. die Polymere, das bzw. die aus amphoteren Polymeren und kationischen Polymeren ausgewählt sind, alleine oder als Mischung ausgewählt sind aus:
- kationischen Polysacchariden, vorzugsweise kationischen Cellulosederivaten, insbesondere kationischen assoziativen Cellulosen, kationischen Derivaten von Cassiagummi, insbesondere denjenigen mit quartären Ammoniumgruppen;
- Homopolymeren oder Copolymeren von Dimethyldiallylammoniumsalzen, vorzugsweise Copolymeren von Dimethyldiallylammoniumsalzen, wie Copolymeren von Diallyldimethylammoniumsalzen (beispielsweise Chlorid) und von Acrylamid;
- amphoteren Polymere, umfassend:
- 30 Mol-% bis 70 Mol-%, noch besser 40 Mol-% bis 60 Mol%, Einheiten, die sich von einem Monomer vom (Meth)acrylamid-Typ (i) ableiten,
- 10 Mol-% bis 60 Mol-%, noch besser 20 Mol-% bis 55 Mol-% Einheiten, die sich von einem Monomer vom (Meth)acrylamidoalkyltrialkylammonium-Typ (ii) ableiten, und
- 1 Mol-% bis 20 Mol-%, noch besser 5 Mol-% bis 15 Mol-% Einheiten, die sich von einem Monomer vom (Meth)acrylsäure-Typ (iii) ableiten;
insbesondere ausgewählt aus Acrylamid/Methacrylamidopropyltrimethylammoniumchlorid/ Acrylsäure-Terpolymeren; insbesondere Polyquaternium 53.

14. Zusammensetzung nach einem der vorhergehenden Ansprüche, umfassend die kationischen und/oder amphoteren Polymere in einer Gesamtmenge im Bereich von 0,01 bis 5 Gew.-%, bezogen auf das Gesamtgewicht der Zusammensetzung, vorzugsweise von 0,05 bis 4 Gew.-%, bevorzugt von 0,1 bis 3 Gew.-% und noch besser von 0,2 bis 2 Gew.-%, bezogen auf das Gesamtgewicht der Zusammensetzung.

15. Zusammensetzung nach einem der vorhergehenden Ansprüche, umfassend Wasser, insbesondere in einem Gesamtgehalt zwischen 50 und 95 Gew.-%, vorzugsweise zwischen 60 und 90 Gew.-% und noch besser zwischen 65 und 85 Gew.-%, bezogen auf das Gesamtgewicht der Zusammensetzung.

16. Kosmetisches Behandlungsverfahren, vorzugsweise Haarbehandlungsverfahren, insbesondere zum Waschen und/oder Konditionieren von Keratinmaterialien, insbesondere menschlichen Keratinfasern wie dem Haar, umfassend das Aufbringen einer Zusammensetzung nach einem der vorhergehenden Ansprüche auf die Materialien, gefolgt von einer fakultativen Einwirkungszeit und/oder Ausspülen und/oder Trocknen.

17. Verwendung der Zusammensetzung nach einem der Ansprüche 1 bis 15 zur kosmetischen Behandlung, insbesondere zum Waschen und/oder Konditionieren, von Keratinmaterialien, insbesondere menschlichen Keratinfasern wie dem Haar.

## Revendications

1. Composition cosmétique, de préférence composition capillaire, comprenant :
(i) un ou plusieurs tensioactifs anioniques choisis parmi les acides alkyl(amido)éther carboxyliques polyoxyalkylénés et leurs sels ;
(ii) un ou plusieurs tensioactifs anioniques non sulfatés, différents du ou des tensioactifs anioniques d'acides alkyl(amido)éther carboxyliques polyoxyalkylénés (i) ;
(iii) un ou plusieurs tensioactifs non ioniques choisis parmi les alkyl(poly)glycosides ;
(iv) un ou plusieurs tensioactifs amphotères ;
(v) un ou plusieurs polymères choisis parmi les polymères amphotères et les polymères cationiques ; la composition ayant un rapport pondéral de la teneur totale en tensioactifs amphotères (iv) sur la teneur totale en tensioactifs anioniques non sulfatés (ii) différents des tensioactifs anioniques d'acides alkyl(amido)éther carboxyliques polyoxyalkylénés (i) supérieur ou égal à 0,5.

2. Composition selon la revendication précédente, **caractérisée en ce que** les acides alkyl(amido)éther carboxyliques polyoxyalkylénés sont choisis parmi ceux de formule (1) :
R₁-(OC₂H₄)ₙ-OCH₂COOA (1)
dans laquelle :
- R1 représente un radical alkyle ou alcényle linéaire ou ramifié en C6-C24, un radical alkyl(C8-C9)phényle, un radical R2CONH-CH2-CH2-, R2 désignant un radical alkyle ou alcényle linéaire ou ramifié en C9-C21 ; de préférence R1 est un radical alkyle en C8-C20, de préférence en C8-C18,
- n est un nombre entier ou décimal variant de 2 à 24, de préférence de 2 à 10,
- A désigne H, ammonium, Na, K, Li, Mg, Ca ou un radical monoéthanolamine ou triéthanolamine.

3. Composition selon l'une quelconque des revendications précédentes, comprenant ledit ou lesdits acides alkyl(amido)éther carboxyliques polyoxyalkylénés et/ou leurs sels, en une quantité totale allant de 0,1 % à 30 % en poids, de préférence de 0,5 % à 25 % en poids, mieux de 1 % à 20 % en poids et préférentiellement de 1,5 % à 10 % en poids par rapport au poids total de la composition.

4. Composition selon l'une des revendications précédentes, dans laquelle les tensioactifs anioniques non sulfatés (ii) sont choisis, seuls ou en mélange, parmi :
- les sulfonates d'oléfines en C6-C24 et notamment en C12-C20 ;
- les alkylsulfosuccinates en C6-C24 et notamment en C12-C20, notamment les laurylsulfosuccinates ;
- les alkyléthersulfosuccinates en C6-C24 et notamment en C12-C20 ;
- les (C6-C24)acyliséthionates, de préférence les (C12-C18)acyliséthionates ;
- les acylsarcosinates en C6-C24 et notamment en C12-C20 ; notamment les palmitoylsarcosinates ;
- les acylglutamates en C6-C24 et notamment en C12-C20 ;
- les acylglycinates en C6-C24 et notamment en C12-C20 ; en particulier sous forme de sels de métaux alcalins ou alcalino-terreux, d'ammonium, ou d'aminoalcool.

5. Composition selon l'une des revendications précédentes, comprenant le ou les tensioactifs anioniques non sulfatés supplémentaires (ii) en une teneur totale allant de 3 % à 20 % en poids, de préférence allant de 3,5 % à 18 %, mieux de 4 % à 15 % en poids, et encore mieux de 5 % à 10 % en poids, par rapport au poids total de la composition.

6. Composition selon l'une quelconque des revendications précédentes, dans laquelle les alkyl(poly)glycosides sont représentés par la formule : R1O-(R2O)t-(G)v dans laquelle :
- R1 représente un radical alkyle ou alcényle linéaire ou ramifié comportant 6 à 24 atomes de carbone et notamment 8 à 18 atomes de carbone, ou un radical alkylphényle dont le radical alkyle linéaire ou ramifié comporte 6 à 24 atomes de carbone et notamment 8 à 18 atomes de carbone,
- R2 représente un radical alkylène comportant 2 à 4 atomes de carbone,
- G représente un motif de sucre comportant 5 à 6 atomes de carbone,
- t désigne une valeur allant de 0 à 10 et de préférence de 0 à 4,
- v désigne une valeur allant de 1 à 15 et de préférence 1 à 4 ;
en particulier sont les (alkyle en C6-C24) (poly)glycosides, et plus particulièrement les (alkyle en C8-C18) (poly)glycosides.

7. Composition selon l'une quelconque des revendications précédentes, comprenant les tensioactifs non ioniques en une teneur totale allant de 0,1 % à 15 % en poids, préférentiellement allant de 0,2 % à 10 % en poids, en particulier allant de 0,5 % à 8 % en poids, par rapport au poids total de la composition.

8. Composition selon l'une quelconque des revendications précédentes, dans laquelle les tensioactifs amphotères sont choisis parmi les alkyl (C₈-C₂₀) bétaïnes, les alkyl(C₈-C₂₀)sulfobétaïnes, les alkyl(C₈-C₂₀)amidoalkyl(C₃-C₈)bétaïnes et les alkyl(C₈-C₂₀)amidoalkyl(C₆-C₈)sulfobétaïnes, ainsi que :
- les composés ayant les structures respectives (II) et (III) ci-dessous :
Rₐ-CONHCH₂CH₂-N⁺(R_{b})(R_{c})-CH₂COO⁻, M⁺, X⁻ (II)
dans laquelle :
- Rₐ représente un groupe alkyle ou alcényle en C₁₀ à C₃₀ dérivé d'un acide Rₐ- COOH, de préférence présent dans l'huile de coprah hydrolysée, ou un groupe heptyle, nonyle ou undécyle ;
- R_{b} représente un groupe β-hydroxyéthyle ; et
- R_{c} représente un groupe carboxyméthyle ;
- M⁺ représente un contre-ion cationique issu d'un métal alcalin ou d'un métal alcalino-terreux, tel que le sodium, un ion ammonium ou un ion issu d'une amine organique ; et
- X⁻ représente un contre-ion anionique organique ou minéral, tel que ceux choisis parmi les halogénures, acétates, phosphates, nitrates, sulfates d'alkyle en (C₁-C₄), (C₁-C₄)alkyl- ou (C₁-C₄)alkylarylsulfonates, en particulier le sulfate de méthyle et le sulfate d'éthyle ; ou en variante M⁺ et X⁻ sont absents ;
R_{a'}-CONHCH₂CH₂-N(B)(B') (III)
dans laquelle :
- B représente le groupe -CH₂CH₂OX' ;
- B' représente le groupe -(CH₂)_{Z}Y', z = 1 ou 2 ;
- X' représente le groupe -CH₂COOH, -CH₂-COOZ', - CH₂CH₂COOH ou CH₂CH₂-COOZ', ou un atome d'hydrogène ;
- Y' représente le groupe -COOH, -COOZ' ou -CH₂CH(OH)SO₃H ou le groupe CH₂CH(OH)SO₃-Z' ;
- Z' représente un contre-ion cationique issu d'un métal alcalin ou d'un métal alcalino-terreux, tel que le sodium, un ion ammonium ou un ion issu d'une amine organique ;
- R_{a'} représente un groupe alkyle ou alcényle en C₁₀ à C₃₀ d'un acide R_{a'}-COOH qui est de préférence présent dans l'huile de coprah ou dans l'huile de lin hydrolysée, ou un groupe alkyle, notamment un groupe en C₁₇, et sa forme iso, ou un groupe en C₁₇ insaturé.
- les composés de formule (IV) : R_{a"}-NHCH(Y")-(CH₂)ₙCONH(CH₂)ₙ-N(R_{d})(Rₑ)
dans laquelle :
- Y" représente le groupe -COOH, -COOZ" ou -CH₂-CH(OH)SO₃H ou le groupe CH₂CH(OH)SO₃-Z" ;
- R_{d} et Rₑ, indépendamment l'un de l'autre, représentent un radical alkyle ou hydroxyalkyle en C₁ à C₄ ;
- Z" représente un contre-ion cationique issu d'un métal alcalin ou d'un métal alcalino-terreux, tel que le sodium, un ion ammonium ou un ion issu d'une amine organique ;
- R_{a"} représente un groupe alkyle ou alcényle en C₁₀ à C₃₀ d'un acide R_{a"}-COOH qui est de préférence présent dans l'huile de coprah ou dans l'huile de lin hydrolysée ;
- n et n', indépendamment l'un de l'autre, désignent un entier allant de 1 à 3.

9. Composition selon l'une quelconque des revendications précédentes, comprenant les tensioactifs amphotères en une teneur totale allant de 3 % à 20 % en poids, préférentiellement en une teneur allant de 3,5 % à 15 % en poids et mieux encore de 4 % à 10 % en poids, par rapport au poids total de la composition.

10. Composition selon l'une quelconque des revendications précédentes, ayant un rapport pondéral de la teneur totale en tensioactifs amphotères (iv) sur la teneur totale en tensioactifs anioniques non sulfatés (ii) différents des tensioactifs anioniques d'acides alkyl(amido)éther carboxyliques polyoxyalkylénés (i) supérieur ou égal à 0,6, en particulier supérieur ou égal à 0,8 ; de préférence compris entre 0,5 et 3,0, notamment entre 0,6 et 2,0, et mieux entre 0,7 et 1,5.

11. Composition selon l'une quelconque des revendications précédentes, dans laquelle la densité de charges cationiques du ou des polymères choisis parmi les polymères amphotères et les polymères cationiques est inférieure ou égale à 4 méq/g.

12. Composition selon l'une quelconque des revendications précédentes, dans laquelle le ou les polymères choisis parmi les polymères amphotères et les polymères cationiques sont choisis parmi :
(1) les homopolymères ou copolymères dérivés d'esters ou d'amides acryliques ou méthacryliques et comportant au moins un des motifs ayant la formule suivante : 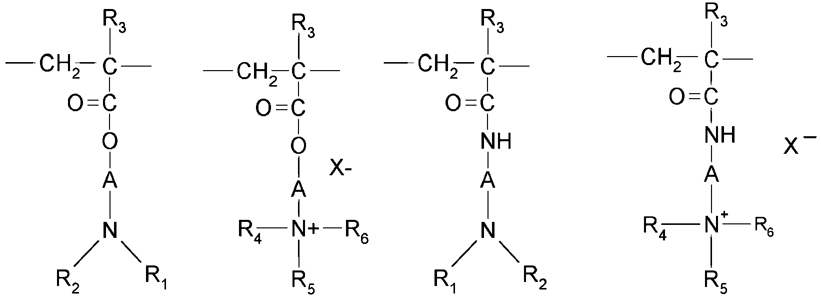 dans laquelle :
- R3, qui peuvent être identiques ou différents, désignent un atome d'hydrogène ou un radical CH3 ;
- A, qui peuvent être identiques ou différents, représentent un groupe divalent alkyle linéaire ou ramifié de 1 à 6 atomes de carbone, de préférence 2 ou 3 atomes de carbone ou un groupe hydroxyalkyle de 1 à 4 atomes de carbone ;
- R4, R5 et R6, qui peuvent être identiques ou différents, représentent un groupe alkyle contenant de 1 à 18 atomes de carbone ou un radical benzyle, de préférence un groupe alkyle contenant de 1 à 6 atomes de carbone ;
- R1 et R2, qui peuvent être identiques ou différents, représentent un atome d'hydrogène ou un groupe alkyle contenant de 1 à 6 atomes de carbone, de préférence méthyle ou éthyle ;
- X désigne un anion dérivé d'un acide minéral ou organique tel qu'un anion méthosulfate ou un halogénure tel que chlorure ou bromure.
(2) les polysaccharides cationiques, notamment les celluloses cationiques associatives ou non associatives et les gommes de galactomannane ; plus particulièrement les dérivés d'éther de cellulose comportant des groupes ammonium quaternaires, les copolymères de cellulose cationique ou de dérivés de cellulose greffés avec un monomère d'ammonium quaternaire hydrosoluble, les gommes de galactomannane cationiques ; les dérivés cationiques de la gomme de cassia, notamment ceux comportant des groupes ammonium quaternaires ; et les celluloses associatives cationiques, en particulier les celluloses quaternisées modifiées par des groupes comportant au moins une chaîne grasse, tels que des groupes alkyle linéaires ou ramifiés, des groupes arylalkyle linéaires ou ramifiés, des groupes alkylaryles linéaires ou ramifiés, de préférence des groupes alkyles linéaires ou ramifiés, ces groupes comportant au moins 8 atomes de carbone, notamment de 8 à 30 atomes de carbone, mieux de 10 à 24 ou même de 10 à 14 atomes de carbone ;
(3) les polymères constitués de motifs pipérazinyle et de radicaux divalents alkylène ou hydroxyalkylène contenant des chaînes linéaires ou ramifiées, éventuellement interrompues par des atomes d'oxygène, de soufre ou d'azote ou par des cycles aromatiques ou hétérocycliques, ainsi que les produits d'oxydation et/ou de quaternisation de ces polymères ;
(4) les polyaminoamides hydrosolubles préparés en particulier par polycondensation d'un composé acide avec une polyamine ; ces polyaminoamides peuvent être réticulés avec une épihalohydrine, un diépoxyde, un dianhydride, un dianhydride insaturé, un dérivé bis-insaturé, une bis-halogénohydrine, un bis-azétidinium, une bis-halogénoacyldiamine, un halogénure de bis-alkyle ou en variante avec un oligomère résultant de la réaction d'un composé difonctionnel qui est réactif avec une bis-halohydrine, un bis-azétidinium, une bis-halogénoacyldiamine, un halogénure de bis-alkyle, une épihalogénohydrine, un diépoxyde ou un dérivé bis-insaturé ; l'agent de réticulation étant utilisé en des proportions allant de 0,025 à 0,35 mol par groupe amine du polyaminoamide ; ces polyaminoamides peuvent être alkylés ou, s'ils comportent une ou plusieurs fonctions amine tertiaire, ils peuvent être quaternisés ;
(5) les dérivés de polyaminoamides résultant de la condensation de polyalkylène polyamines avec des acides polycarboxyliques suivie d'une alkylation avec des agents difonctionnels ;
(6) les polymères obtenus par mise en réaction d'une polyalkylène polyamine comportant deux groupes amine primaire et au moins un groupe amine secondaire avec un acide dicarboxylique choisi parmi l'acide diglycolique et les acides dicarboxyliques aliphatiques saturés contenant de 3 à 8 atomes de carbone ;
(7) les cyclopolymères d'alkyldiallylamine ou de dialkyldiallylammonium tels que les homopolymères ou copolymères comportant comme constituant principal de la chaîne, des motifs correspondant aux formules (I) ou (II) : 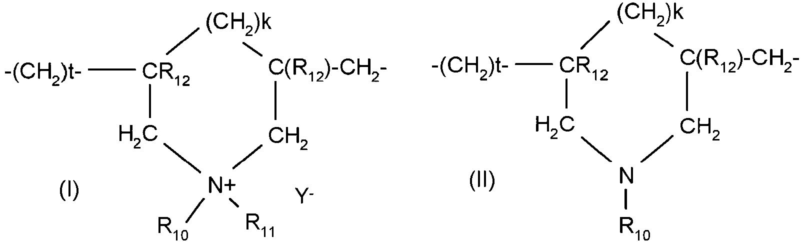 dans lesquelles
- k et t sont égaux à 0 ou 1, la somme k + t étant égale à 1 ;
- R12 désigne un atome d'hydrogène ou un radical méthyle ;
- R10 et R11, indépendamment l'un de l'autre désignent un groupe alkyle en C1-C6, un groupe hydroxyalkyle en C1-C5, un groupe amidoalkyle en C1-C4 ; ou en variante R10 et R11 peuvent désigner conjointement avec l'atome d'azote auquel ils sont fixés un groupe hétérocyclique tel que pipéridinyle ou morpholinyle ; R10 et R11, indépendamment l'un de l'autre, de préférence désignent un groupe alkyle en C1-C4 ;
- Y⁻ est un anion tel que bromure, chlorure, acétate, borate, citrate, tartrate, bisulfate, bisulfite, sulfate ou phosphate ;
(8) les polymères de diammonium quaternaire comprenant des motifs répétitifs de formule : 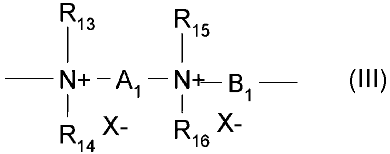 dans laquelle :
- R13, R14, R15 et R16, qui peuvent être identiques ou différents, représentent des radicaux aliphatiques, alicycliques, ou arylaliphatiques comprenant de 1 à 20 atomes de carbone ou des radicaux hydroxyalkylaliphatiques en C1-C12,
ou bien R13, R14, R15 et R16, conjointement ou séparément, forment, avec les atomes d'azote auxquels ils sont fixés, des hétérocycles comprenant éventuellement un second hétéroatome autre que l'azote ;
ou bien R13, R14, R15 et R16 représentent un radical alkyle en C1-C6 linéaire ou ramifié substitué par un groupe nitrile, ester, acyle, amide ou -CO-O-R17-D ou - CO-NH-R17-D où R17 est un alkylène et D est un groupe ammonium quaternaire ;
- A1 et B1 représentent des groupes polyméthylène divalents, linéaires ou ramifiés, saturés ou non saturés, comprenant de 2 à 20 atomes de carbone, qu'ils peuvent contenir, liés à ou intercalés dans la chaîne principale, un ou plusieurs cycles aromatiques, ou un ou plusieurs atomes d'oxygène, de soufre ou groupes sulfoxyde, sulfone, disulfure, amino, alkylamino, hydroxyle, ammonium quaternaire, uréido, amide ou ester, et
- X⁻ désigne un anion dérivé d'un acide minéral ou organique ;
étant entendu que A1, R13 et R15 peuvent former avec les deux atomes d'azote auxquels ils sont fixés, un cycle pipérazine ;
en outre, si A1 désigne un radical alkylène ou hydroxyalkylène linéaire ou ramifié, saturé ou non saturé, B1 peut également désigner un groupe (CH2)n-CO-D-OC-(CH2)p-, n et p, qui peuvent être identiques ou différents, étant des entiers allant de 2 à 20, et D désignant :
a) un radical glycol de formule -O-Z-O-, dans laquelle Z désigne un radical à base d'hydrocarbure linéaire ou ramifié ou un groupe correspondant à l'une des formules suivantes : -(CH2CH2O)x-CH2CH2- et -[CH2CH(CH3)O]y-CH2CH(CH3)-, dans lesquelles x et y désignent un entier de 1 à 4, représentant un degré de polymérisation défini et unique ou un nombre quelconque de 1 à 4 représentant un degré de polymérisation moyen ;
b) un radical de diamine bis-secondaire tel qu'un dérivé de pipérazine ;
c) un radical de diamine bis-primaire de formule -NH-Y-NH- dans laquelle Y désigne un radical à base d'hydrocarbure linéaire ou ramifié, ou bien le radical divalent -CH2-CH2-S-S-CH2-CH2- ;
d) un groupe uréylène de formule -NH-CO-NH-.
(9) les polymères d'ammonium polyquaternaires comprenant des motifs de formule (V) : 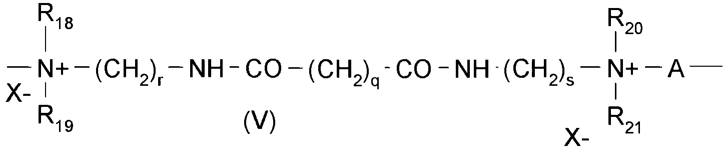 dans laquelle :
- R18, R19, R20 et R21, qui peuvent être identiques ou différents, représentent un atome d'hydrogène ou un radical méthyle, éthyle, propyle, β-hydroxyéthyle, β-hydroxypropyle ou - CH2CH2(OCH2CH2)pOH, dans laquelle p est égal à 0 ou à un entier compris entre 1 et 6, sous réserve que R18, R19, R20 et R21 ne représentent pas simultanément un atome d'hydrogène,
- r et s, qui peuvent être identiques ou différents, sont des entiers compris entre 1 et 6,
- q est égal à 0 ou à un entier compris entre 1 et 34,
- X- désigne un anion tel qu'un halogénure,
- A désigne un radical dihalogénure divalent ou représente de préférence -CH2-CH2-O-CH2-CH2- ;
(10) les polymères quaternaires de vinylpyrrolidone et de vinylimidazole ;
(11) les polyamines ;
(12) les polymères comportant dans leur structure :
(a) un ou plusieurs motifs correspondant à la formule (A) ci-dessous : 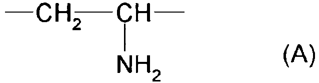
(b) éventuellement un ou plusieurs motifs correspondant à la formule (B) ci-dessous : 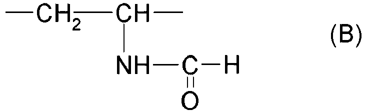
(13) les polymères amphotères comprenant une répétition de :
(i) un ou plusieurs motifs issus d'un monomère de type (méth)acrylamide,
(ii) un ou plusieurs motifs issus d'un monomère de type (méth)acrylamidoalkyl-trialkylammonium, et
(iii) un ou plusieurs motifs issus d'un monomère d'acide de type acide (méth)acrylique.

13. Composition selon l'une quelconque des revendications précédentes, dans laquelle le ou les polymères choisis parmi les polymères amphotères et les polymères cationiques, sont choisis, seuls ou en mélange, parmi :
- les polysaccharides cationiques, préférentiellement les dérivés de cellulose cationiques, notamment les celluloses associatives cationiques, les dérivés cationiques de gomme de cassia, notamment ceux comportant des groupes ammonium quaternaires ;
- les homopolymères ou copolymères de sels de diméthyldiallylammonium, préférentiellement les copolymères de sels de diméthyldiallylammonium, tels que les copolymères de sels (par exemple chlorure) de diallyldiméthylammonium et d'acrylamide ;
- les polymères amphotères comprenant :
- de 30 % en moles à 70 % en moles, mieux de 40 % en moles à 60 % en moles, de motifs issus d'un monomère de type (méth)acrylamide (i),
- de 10 % en moles à 60 % en moles, mieux de 20 % en moles à 55 % en moles, de motifs issus d'un monomère de type (méth)acrylamidoalkyltrialkylammonium (ii), et
- de 1 % en moles à 20 % en moles, mieux de 5 % en moles à 15 % en moles, de motifs issus d'un monomère de type acide (méth)acrylique (iii) ;
notamment choisis parmi les terpolymères acrylamide/chlorure de méthacrylamidopropyltriméthylammonium/acide acrylique ; en particulier le Polyquaternium 53.

14. Composition selon l'une quelconque des revendications précédentes, comprenant lesdits polymères cationiques et/ou amphotères en une quantité totale allant de 0,01 % à 5 % en poids par rapport au poids total de la composition, de préférence de 0,05 % à 4 % en poids, préférentiellement de 0,1 % à 3 % en poids et encore mieux de 0,2 % à 2 % en poids, par rapport au poids total de la composition.

15. Composition selon l'une quelconque des revendications précédentes, comprenant de l'eau, notamment en une teneur totale comprise entre 50 % et 95 % en poids, de préférence entre 60 % et 90 %, et mieux entre 65 % et 85 % en poids par rapport au poids total de la composition.

16. Procédé de traitement cosmétique, de préférence procédé de traitement capillaire, notamment pour le lavage et/ou le conditionnement de matières kératiniques, en particulier des fibres kératiniques humaines telles que les cheveux, comprenant l'application sur lesdites matières d'une composition selon l'une des revendications précédentes, suivie par un éventuel temps de pose et/ou d'un rinçage et/ou d'un séchage.

17. Utilisation de la composition selon l'une des revendications 1 à 15, pour le traitement cosmétique, en particulier pour le lavage et/ou le conditionnement de matières kératiniques, en particulier des fibres kératiniques humaines telles que les cheveux.
